# EUROPEAN PATENT APPLICATION

(11) **EP 1 468 999 A1**
(43) Date of publication of application: **20.10.2004**
(21) Application number: 03006256.6
(22) Date of filing: 20.03.2003
(51) Int. Cl.: C07D 471/10, C07D 401/12, C07D 521/00, C07D 211/62, C07D 295/12, A61K 31/451, A61K 31/4725, A61P 3/04, A61P 15/00, A61P 25/22, A61P 25/24

(54) **Substituted piperidine and piperazine derivatives as melanocortin-4 receptor modulators**

(71) Applicant: MyoContract Ltd., 4410 Liestal (CH)
(72) Inventor: Soeberdt, Michael, 79618 Rheinfelden (DE); Weyermann, Philipp, 4450 Sissach (CH); Sprecher von, Andreas, 4104 Oberwill (CH); Henneböhle, Marco, 79618 Rheinfelden (DE)
(74) Representative: Grünecker, Kinkeldey, Stockmair & Schwanhäusser Anwaltssozietät

(57) **Abstract**

The present invention relates to novel substituted piperidine and piperazine derivatives as melanocortin-4 receptor (MC-4R) modulators. MC-4R agonists of the invention can be used for the treatment of disorders and diseases such as obesity, diabetes and sexual dysfunction, whereas the MC-4R antagonists are useful for the treatment of disorders and diseases such as cancer cachexia, muscle wasting, anorexia, anxiety and depression. All diseases and disorders, where the regulation of the MC-4R is involved, can be treated with the compounds of the invention.

## Description

### Field of the Invention

The present invention relates to novel substituted piperidine and piperazine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly, α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-R's results in stimulation of CAMP formation.

To date, five distinct types of receptor subtype for MC (MC-1R to MC-5R) have been identified and these are expressed in different tissues.

MC-1 R was first found in melanocytes. Naturally occurring inactive variants of MC-1 R in animals were shown to lead to alterations in pigmentation and a subsequent lighter coat color by controlling the conversion of phaeomelanin to eumelanin through the control of tyrosinase. From these and other studies, it is evident that MC-1 R is an important regulator of melanin production and coat color in animals and skin color in humans.

The MC-2R is expressed in the adrenal gland representing the ACTH receptor. The MC-2R is not a receptor for α-MSH but is the receptor for the adrenocorticotropic hormone I (ACTH l).

The MC-3R is expressed in the brain (predominately located in the hypothalamus) and peripheral tissues like gut and placenta, and knock-out studies, have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask, et al., "Selective antagonist for the melanocortin-4 receptor (HS014) increases food intake in free-feeding rats", Biochem. Biophys. Res. Commun., 245: 90-93 (1998)).

MC-5R is ubiquitously expressed in many peripheral tissues including white fat and placenta, and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators, to near baseline levels. Recent studies have demonstrated that the anti-inflammatory actions of α-MSH are mediated by MC-1 R. The mechanism by which agonism of MC-1 R results in an anti-inflammatory response is likely through inhibition of the pro-inflammatory transcription activator, NF-κB. NF-κB is a pivotal component of the pro-inflammatory cascade and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands", Brain Research, 80: 302-306 (1998)). Further evidence for the involvement of MC-R's in obesity includes: 1) the agouti (A^{vy}) mouse, which ectopically expresses an antagonist of the MC-1 R, MC-3R and MC-4R, is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R and -5R agonist) injected intracerebroventricularly (ICV) in rodents, reduces food intake in several animal feeding models (NPY, ob/ob, agouti and fasted) while ICV injected SHU-9119 (MC-3R and -4R antagonist; MC-1R and -5R agonist) reverses this effect and can induce hyperphagia; 4) chronic intraperitoneal treatment of Zucker fatty rats with an α-NDP-MSH derivative (HP-228) has been reported to activate MC-1 R, -3R, -4R and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study", J Urol., 160: 389-393, 1998). Activation of melanocortin receptors of the brain appears to cause normal stimulation of sexual arousal. Evidence for the involvement of MC-R in male and/or female sexual dysfunction is detailed in WO/0074679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin-responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin-sensitive cells, insulin-insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF), (Owen MJ and Nemeroff CB (1991) Physiology and pharmacology of corticotrophin releasing factor. *Pharmacol Rev* 43:425-473). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Anxiolytic-Like and Antidepressant-Like Activities of MCL0129 (1-[(S)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor, Shigeyuki Chaki et al, J. Pharm. Exp. Ther. (2003)304(2), 818-26).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus, by α-MSH, reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (Marks D.L. *et al.* Role of the central melanocortin system in cachexia. Cancer Res. (2001) 61: 1432-1438)

In view of the unresolved deficiencies in treatment of various diseases and disorders as discussed above, it is an object of the present invention to provide novel substituted piperidine and piperazine derivatives with improved ability to cross the blood brain barrier, which are useful as melanocortin-4 receptor modulators to treat cancer cachexia, muscle wasting, anorexia, anxiety, depression, obesity, diabetes, sexual dysfunction and other diseases with MC-4R involvement.

### Summary of the Invention

The present invention relates to novel substituted piperidine and piperazine derivatives of structural formula (I), 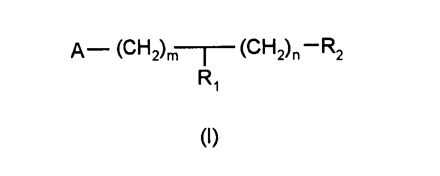 wherein the variables A, R₁, R₂, m and n have the meaning as defined below.

The piperidine and piperazine derivatives of structural formula (I) are effective as melanocortin receptor modulators and are particularly effective as selective melanocortin-4 receptor (MC-4R) modulators. They are therefore useful for the treatment of disorders where the activation or inactivation of the MC-4R are involved. Agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression.

The present invention also relates to pharmaceutical compositions comprising the compounds of the present invention and a pharmaceutically acceptable carrier.

### Detailed Description of the Invention

The present invention relates to novel substituted piperidine and piperazine derivatives useful as melanocortin receptor modulators, in particular, selective MC-4R agonists and MC-4R antagonists.

The compounds of the present invention are represented by structural formula (I), 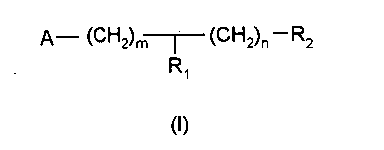 or a pharmaceutically acceptable salt or a solvate thereof, wherein
- R₁: is:
(D)-aryl or
(D)-heteroaryl,
wherein aryl and heteroaryl are unsubstituted or substituted;
- R₂: is: 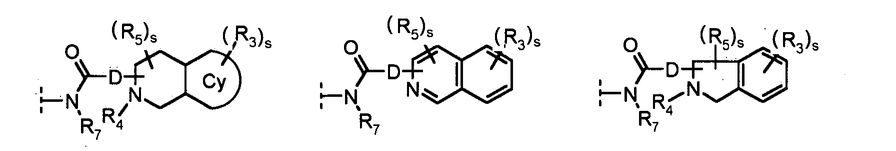 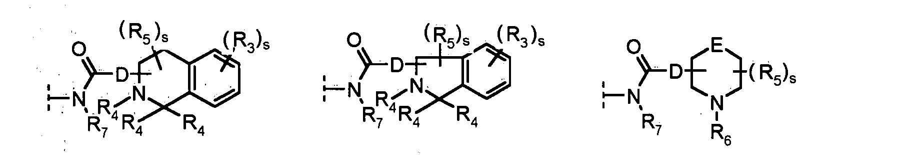 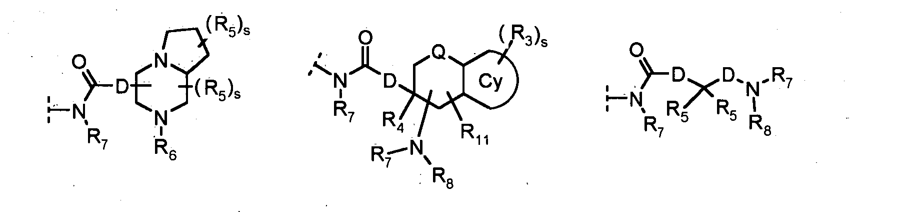 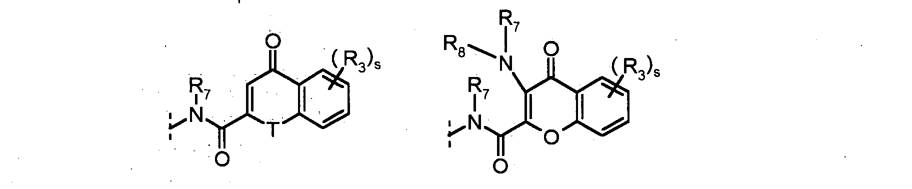 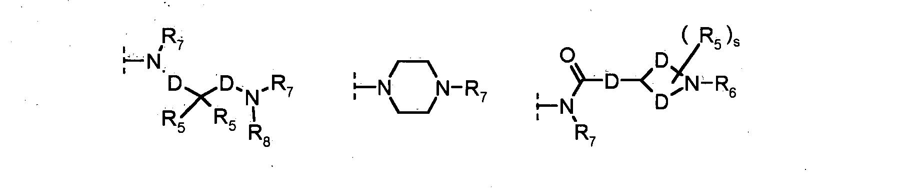
- A: is: 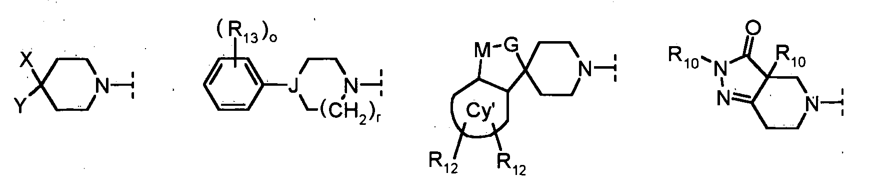
- each R₃: is independently:
hydrogen,
halo,
alkyl,
haloalkyl,
(D)-cycloalkyl,
(D)-aryl (wherein aryl being phenyl or naphthyl),
(D)-heteroaryl,
(D)-heterocyclyl (wherein heterocyclyl excludes a heterocyclyl containing a single nitrogen), and
wherein aryl, heteroaryl, heterocyclyl, alkyl and cycloalkyl is unsubstituted or substituted;
- each R₄: is independently:
hydrogen,
alkyl,
C(O)-alkyl,
SO₂alkyl,
SO₂aryl,
(D)-aryl or
(D)-cycloalkyl;
- each R₅: is independently:
hydrogen,
alkyl,
(D)-aryl,
(D)-heteroaryl,
(D)-N(R₇)₂,
(D)-NR₇C(O)-alkyl,
(D)-NR₇SO₂-alkyl,
(D)-SO₂N(R₇)₂,
(D)-(O)_{q}-alkyl,
(D)-(O)_{q}(D)-NR₇COR₇,
(D)-(O)_{q}(D)-NR₇SO₂R₇,
(D)-(O)_{q}-heterocyclyl or
(D)-(O)_{q}(alkyl)-heterocyclyl;
- each R₆: is independently:
hydrogen,
alkyl,
(D)-phenyl,
C(O)-alkyl,
C(O)-phenyl,
SO₂-alkyl or
SO₂-phenyl;
- R₇ and R₈: are each independently:
hydrogen,
alkyl or
(D)-cycloalkyl, or
R₇ and R₈ together with the nitrogen to which they are attached form a 5- to 8-membered ring optionally containing an additional heteroatom selected from O, S and NR₄,
wherein alkyl and cycloalkyl are unsubstituted or substituted;
- R₁₀: is independently:
hydrogen,
alkyl,
(D)-aryl or
(D)-cycloalkyl;
- R₁₁: is:
hydrogen or
alkyl;
- R₁₂: is:
hydrogen,
halo,
alkyl,
alkoxy,
C=N,
CF₃ or
OCF₃;
- R₁₃: is independently:
hydrogen,
hydroxy,
cyano,
nitro,
halo,
alkyl,
alkoxy,
haloalkyl,
(D)-C(O)R₁₅,
(D)-C(O)OR₁₅,
(D)-C(O)SR₁₅,
(D)-C(O)-heteroaryl,
(D)-C(O)-heterocyclyl,
(D)-C(O)N(R₁₅)₂,
(D)-N(R₁₅)₂,
(D)-NR₁₅COR₁₅,
(D)-NR₁₅CON(R₁₅)₂,
(D)-NR₁₅C(O)OR₁₅,
(D)-NR₁₅C(R₁₅)=N(R₁₅),
(D)-NR₁₅C(=NR₁₅)N(R₁₅)₂,
(D)-NR₁₅SO₂R₁₅,
(D)-NR₁₅SO₂N(R₁₅)2,
(D)-NR₁₅(D)-heterocyclyl,
(D)-NR₁₅(D)-heteroaryl,
(D)-OR₁₅,
OSO₂R₁₅,
(D)-[O]_{q}(cycloalkyl),
(D)-[O]_{q}(D)-aryl,
(D)-[O]_{q}(D)-heteroaryl,
(D)-[O]_{q}(D)-heterocyclyl,
(D)-SR₁₅,
(D)-SOR₁₅,
(D)-SO₂R₁₅ or
(D)-SO₂N(R₁₅)₂,
wherein alkyl, alkoxy, cycloalkyl, aryl, heterocyclyl and heteroaryl are unsubstituted or substituted;
- each R₁₅: is independently:
hydrogen,
alkyl,
haloalkyl,
(D)-cycloalkyl,
(D)-aryl (wherein aryl is phenyl or naphthyl),
(D)-heteroaryl,
(D)-heterocyclyl (wherein heterocyclyl excludes a heterocyclyl containing a single nitrogen), and
wherein aryl, heteroaryl, heterocyclyl, alkyl and cycloalkyl is unsubstituted or substituted;
- R₁₇: is independently:
R₁₀ or
(D)-heterocyclyl;
- R₁₈: is independently:
R₁₀,
(D)-heteroaryl,
(D)-heterocyclyl,
(D)-N(Y)₂,
(D)-NH-heteroaryl and
(D)-NH-heterocyclyl,
wherein aryl, heteroaryl, alkyl, D, cycloalkyl and heterocyclyl are unsubstituted or substituted, or
two R₁₈ groups together with the atoms to which they are attached form a 5- to 8-membered mono- or bi-cyclic ring system optionally containing an additional heteroatom selected from O, S, NR₁₀, NBoc and NZ;
- Cy: is:
aryl,
5- or 6-membered heteroaryl,
5- or 6-membered heterocyclyl or
5- or 7-membered carbocyclyl;
- Cy': is benzene, pyridine or cyclohexane;
- X: is:
alkyl,
(D)-cycloalkyl,
(D)-aryl,
(D)-heteroaryl,
(D)-heterocyclyl,
(D)-C≡N,
(D)-CON(R₁₇R₁₇),
(D)-CO₂R₁₇,
(D)-COR₁₇.
(D)-NR₁₇C(O)R₁₇.
(D)-NR₁₇CO₂R₁₇,
(D)-NR₁₇C(O)N(R₁₇)₂.
(D)-NR₁₇SO₂R₁₇,
(D)-S(O)ₚR₁₇,
(D)-SO₂N(R₁₇)(R₁₇).
(D)-OR₁₇,
(D)-OC(O)R₁₇,
(D)-OC(O)OR₁₇,
(D)-OC(O)N(R₁₇)₂.
(D)-N(R₁₇)(R₁₇) or
(D)-NR₁₇SO₂N(R₁₇)(R₁₇).
wherein aryl, heteroaryl, alkyl, D, cycloalkyl and heterocyclyl are unsubstituted or substituted;
- Y: is:
hydrogen,
alkyl,
(D)-cycloalkyl,
(D)-aryl,
(D)-heterocyclyl or
(D)-heteroaryl,
wherein aryl, heteroaryl, alkyl, D and cycloalkyl are unsubstituted or substituted;
Q is a bond, O, S(O)ᵤ, NR₆ or CH₂;
D is a bond or C₁ - C₄-alkyl;
E is 0, S or NR₆;
G is D, CH-alkyl, O, C=O or SO₂, with the proviso that when G is O, the ring atom M is carbon;
J is N or CH;
M is CHCO₂Y, CHC(O)N(Y)₂, NSO₂R₁₈, CHN(Y)COR₁₈, CHN(Y)SO₂R₁₈, CHCH₂OY or CHCH₂heteroaryl;
T is O or NR₇;
n is 0 - 3;
m is 1 - 3;
o is 0 - 3;
p is 0 - 2;
q is 0 or 1;
r is 1 or 2;
s is 0 - 3;
u is 0 - 2.

In preferred embodiments, the variants have the following meanings:

R₁ is as defined above and is preferably (D)-aryl, more preferably (D)-phenyl or (D)-naphthyl. If aryl or heteroaryl is substituted, it is preferably substituted with one to three, more preferably 1 or 2, most preferably 1, substituents. The substituents are preferably independently selected from the group consisting of cyano, nitro, perfluoroalkoxy, halo, alkyl, (D)-cycloalkyl, alkoxy, hydroxyl and haloalkyl, more preferably selected from perfluoroalkoxy, halo, alkyl, alkoxy or haloalkyl, even more preferably selected from halo, alkyl, alkoxy and haloalkyl, in particular halo.

Most preferably, R₁ is (CH₂)-phenyl or (CH₂)-naphthyl which both, preferably phenyl, may be substituted with one to three, in particular one halo, e.g. Cl. The substitution can be in any position, preferably in the 4-position.

R₂ is as defined above, preferably 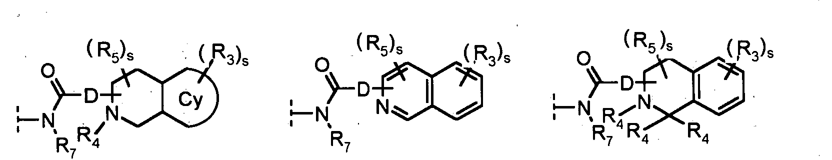 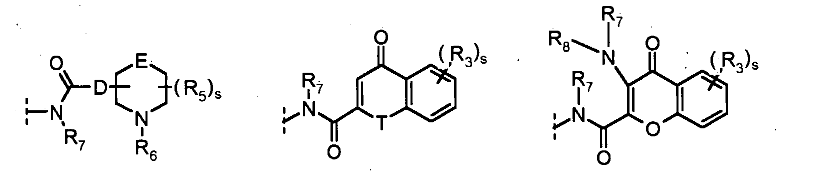 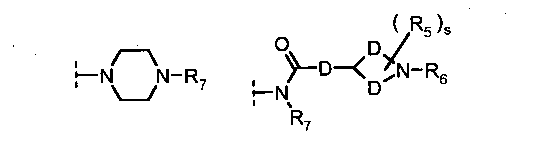 more preferably 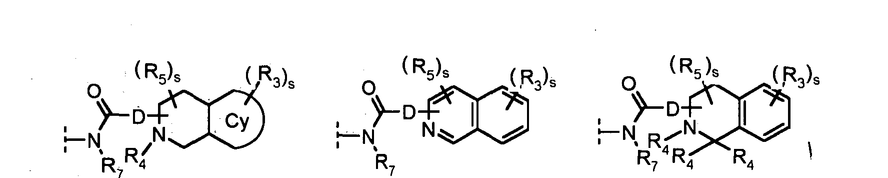 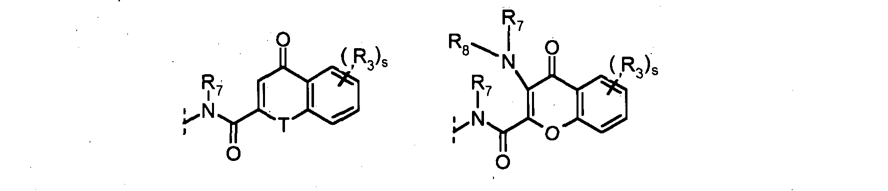 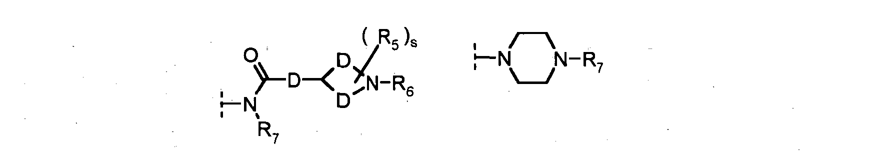 In one embodiment R₂ is

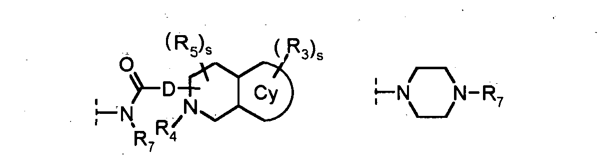

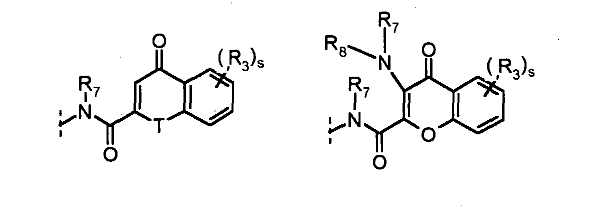

R₃ is as defined above. If aryl, heteroaryl, heterocyclyl, alkyl and/or cycloalkyl are substituted, they are independently preferably substituted with one to three, more preferably one, substituents selected from the group consisting of oxo, alkyl, N(R₄)₂, OR₄, SR₄ and CO₂R₄.

R₃ is preferably hydrogen, halo, unsubstituted alkyl, substituted alkyl, haloalkyl, alkoxy, unsubstituted (D)-cycloalkyl or substituted (D)-cycloalkyl, more preferably hydrogen.

R₄ is as defined above, preferably hydrogen or alkyl, more preferably hydrogen.

R₅ is as defined above, preferably hydrogen, alkyl, (D)-aryl, (D)-heteroaryl, (D)-N(R₇)₂, (D)-NR₇C(O)alkyl or (D)-NR₇SO₂alkyl, more preferably hydrogen.

R₇ and R₈ are each independently as defined above. When R₇ and R₈ form a ring, said ring may contain an additional heteroatom, preferably selected from O, S and NR₄ in the ring. Moreover, if alkyl and cycloalkyl are substituted, they are preferably substituted with one to three, more preferably one or two, groups independently selected from R₉ and oxo.

R₇ and R₈ are each independently preferably selected from the group consisting of hydrogen, alkyl or cycloalkyl, or R₇ and R₈ together with the nitrogen to which they are attached form a 5- to 7-membered ring. More preferably R₇ and R₈ are each independently selected from the group consisting of hydrogen or alkyl, or R₇ and R₈ together with the nitrogen to which they are attached form a 5- to 6-membered ring, optionally containing an additional oxygen atom.

The above mentioned R₉ is alkyl, (D)-aryl, (D)-cycloalkyl, (D)-heteroaryl, halo, OR₁₀, NHSO₂R₁₀, N(R₁₀)₂, C=N, CO₂R₇, C(R₁₀)(R₁₀)N(R₁₀)₂, nitro, SO₂N(R₁₀)₂, S(O)ᵤR₁₀, CF₃ and OCF₃.R₉ is preferably selected from the group consisting of alkyl, OR₁₀, (D)-aryl, (D)-cycloalkyl, (D)-heteroaryl and halo.

R₁₂ is defined as above, preferably hydrogen, halo, alkyl, alkoxy or C≡N, more preferably hydrogen, halo or C₁ - C₄-alkyl, most preferably hydrogen.

R₁₃ is as defined above, wherein heterocyclyl includes azetidin-2-one-1-yl, pyrrolidin-2-one-1-yl, piperid-2-one-1-yl and azepan-2-one-1-yl. Moreover, alkyl, alkoxy, cycloalkyl, aryl, heterocyclyl and heteroaryl are preferably substituted or unsubstituted alkyl with one to five, preferably 1 to 3, more preferably 1 or 2, substituents independently selected from R₁₄. Preferably, R₁₃ is hydrogen, hydroxy, cyano, nitro, halo, alkyl, alkoxy, haloalkyl, (D)-N(R₁₅)₂, (D)-NR₁₅COR₁₅, (D)-NR₁₅CON(R₁₅)₂, (D)-NR₁₅C(O)OR₁₅, (D)-NR₁₅C(R₁₅)=N(R₁₅), (D)-NR₁₅C(=NR₁₅)N(R₁₅)₂, (D)-NR₁₅SO₂R₁₅ or (D)-NR₁₅SO₂N(R₁₅)₂, wherein alkyl or alkoxy are substituted or unsubstituted with one to five, preferably one to three, substituents selected from R₁₄. More preferably, R₁₃ is cyano, nitro, halo, alkyl, (D)-N(R₁₅)₂, (D)-NR₁₅COR₁₅, (D)-NR₁₅CON(R₁₅)₂, (D)-NR₁₅C(O)OR₁₅ or (D)-NR₁₅SO₂R₁₅. Most preferably, R₁₃ is cyano, nitro, halo or (D)-NR₁₅COR₁₅. Halo is preferably F, Cl or Br. R₁₃ can be on any position of the ring, preferably in the 1-position.

R₁₄ is independently, hydrogen, halo, oxo, N(R₁₆)₂, alkyl, (D)-cycloalkyl, haloalkyl, alkoxy, heteroaryl, hydroxy or heterocyclyl, wherein heterocyclyl excludes a heterocyclyl containing a single nitrogen, phenyl, (D)-COR₁₅, (D)-C(O)OR₁₅, (D)-OR₁₅, (D)-OCOR₁₅, (D)-OCO₂R₁₅, (D)-SR₁₅, (D)-SOR₁₅ or (D)-SO₂R₁₅; wherein aryl, heteroaryl, heterocyclyl, alkyl or cycloalkyl are unsubstituted or substituted with one to three substituents selected from the group consisting of oxo, alkyl, N(R₁₆)₂, OR₁₆, SR₁₆ and CO₂R₁₆. Preferably R₁₄ is as defined above, wherein aryl, heteroaryl, heterocyclyl, alkyl or cycloalkyl are preferably substituted or unsubstituted with one to three, more preferably one or two, substituents selected from the group consisting of oxo, alkyl, N(R₁₆)₂, OR₁₆, SR₁₆ and CO₂R₁₆. Preferably, R₁₄ is hydrogen, halo, alkyl, (D)-cycloalkyl, alkoxy or phenyl, more preferably R₁₄ is hydrogen, halo, alkyl, alkoxy or phenyl.

R₁₅ is as defined above wherein aryl, heteroaryl, heterocyclyl, alkyl or cycloalkyl are preferably substituted or unsubstituted with one to three, more preferably one or two, substituents selected from the group consisting of oxo, alkyl, N(R₁₆)₂, OR₁₆, SR₁₆ and CO₂R₁₆. Preferably, R₁₅ is hydrogen, halo, alkyl, (D)-cycloalkyl, alkoxy or phenyl, more preferably R₁₅ is hydrogen, halo, alkyl, alkoxy or phenyl.

Each R₁₆ is hydrogen, alkyl, C(O)-alkyl, aryl or cycloalkyl, preferably hydrogen, alkyl or cycloalkyl, in particular, hydrogen

X is as defined above, wherein aryl and heteroaryl are preferably unsubstituted or substituted with one to three, preferably one or two, groups selected from R₉. Moreover, alkyl, D, cycloalkyl and heterocyclyl are preferably unsubstituted or substituted with one to four groups independently selected from R₉ and oxo. Preferably, X is alkyl, (D)-cycloalkyl, (D)-aryl, (D)-heteroaryl, (D)-heterocyclyl, (D)-NHC(O)R₁₇, (D)-CO₂R₁₇ or (D)-CON(R₁₇R₁₇), more preferably alkyl, (D)-cycloalkyl, (D)-heterocyclyl, (D)-NHC(O)R₁₇ or (D)-CON(R₁₇R₁₇), most preferably C₁ - C₄-alkyl, C₅ - C₇-cycloalkyl, (D)-CON(R₁₇R₁₇) and N-containing heterocyclyl, in particular triazolyl and tetrazolyl.

Y is as defined above, wherein aryl and heteroaryl are preferably unsubstituted or substituted with one to three, preferably one or two, groups selected from R₉. Moreover, alkyl, D and cycloalkyl are preferably unsubstituted or substituted with one to three groups selected from R₉ and oxo. Preferably, Y is hydrogen, alkyl, (D)-cycloalkyl, (D)-aryl, (D)-heteroaryl or (D)-heterocyclyl, more preferably hydrogen, alkyl, (D)-cycloalkyl or (D)-heterocyclyl, most preferably hydrogen, C₁- C₄-alkyl and C₅ - C₇-cycloalkyl, in particular cyclohexyl and phenyl.

Cy is as defined above and preferably selected from aryl, 5- or 6-membered heteroaryl, 5-or 6-membered heterocyclyl and 5- to 7-membered carbocyclyl, more preferably Cy is aryl and heteroaryl. In one embodiment, Cy may be aryl, such as phenyl or naphthyl.

Cy' is as defined above, preferably benzene or pyridine, more preferably benzene.

D is as defined above, preferably a bond or C₁ - C₄-alkylene, more preferably a bond or CH₂.

M is as defined above, preferably NSO₂R₁₈, CHN(Y)COR₁₈ or CHN(Y)SO₂R₁₈, more preferably NSO₂R₁₈

G is as defined above, preferably D or CH-alkyl, more preferably D, in particular CH₂.

J is N or CH;
T is O or NR₇, preferably NR₇;
n is 0, 1 or 2, preferably 0 or 1;
m is 1, 2 or 3, preferably 1 or 2;
p is 0, 1 or 2;
q is 0 or 1,
r is 1 or 2, preferably 1;
s is 0, 1, 2 or 3, preferably 0, 1 or 2, more preferably 0 or 1.

In the above and the following, the employed terms have the meaning as described below:

Aryl is an aromatic mono- or polycyclic moiety with 6 to 20 carbon atoms which is preferably selected from phenyl, biphenyl, naphthyl, tetrahydronaphthyl, fluorenyl, indenyl or phenanthrenyl, more preferably phenyl or naphthyl.

Heteroaryl is an aromatic moiety having 6 to 20 carbon atoms with at least one heterocycle and is preferably selected from thienyl, benzothienyl, naphthothienyl, furanyl, benzofuranyl, chromenyl, indolyl, isoindolyl, indazolyl, quinolyl, isoquinolyl, phthalazinyl, quinoxalinyl, cinnolinyl or quinazolinyl, more preferably thienyl, furanyl, benzothienyl, benzofuranyl or indolyl.

Heterocyclyl is a saturated, unsaturated or aromatic ring containing at least one heteroatom selected from O, N and/or S and 1 to 6 carbon atoms, and is preferably selected from azetidin-2-one-1-yl, pyrrolidin-2-one-1-yl, piperid-2-one-1-yl, azepan-2-one-1-yl, thienyl, furyl, piperidinyl, pyranyl, pyrrolyl, imidazolyl, pyrazolyl, pyridyl, pyrazinyl, pyrimidinyl, pyridazinyl, triazolyl, isothiazolyl or isoxazyl, more preferably pyridyl, piperidinyl, triazolyl, imidazolyl or pyrazinyl.

Carbocyclyl is a monocyclic or polycyclic ring system of 3 to 20 carbon atoms which may be saturated, unsaturated or aromatic.

Alkyl is a straight chain or a branched alkyl having preferably 1 to 8 carbon atoms, such as methyl, ethyl, n-propyl, isopropyl, n-butyl, isobutyl, tert-butyl, n-pentyl, isopentyl, neopentyl, hexyl or heptyl, more preferably 1 to 4 carbon atoms.

Cycloalkyl is an alkyl ring having preferably 3 to 8 carbon atoms, such as cyclopropyl, cyclobutyl, cyclopentyl, cyclohexyl, cycloheptyl or cyclooctyl, more preferably 3 to 6 carbon atoms.

Alkoxy is O-alkyl, wherein alkyl is as defined above and has preferably 1 to 4 carbon atoms, preferably 1 or 2 carbon atoms.

Halo or halogen is a halogen atom preferably selected from F, Cl, Br and I, preferably F, Cl and Br.

Haloalkyl is an alkyl moiety as defined above having preferably 1 to 4 carbon atoms, more preferably 1 or 2 carbon atoms, wherein at least one, preferably 1 to 3 hydrogen atoms have been replaced by a halogen atom. Preferred examples are -CF₃, -CH₂CF₃ -CF₂CF₃.

Therein, the alkylene moiety may be a straight chain or branched chain group. Said alkylene moiety preferably has 1 to 6 carbon atoms. Examples thereof include methylene, ethylene, n-propylene, n-butylene, n-pentylene, n-hexylene, iso-propylene, sec.-butylene, tert.-butylene, 1,1-dimethyl propylene, 1,2-dimethyl propylene, 2,2-dimethyl propylene, 1,1-dimethyl butylene, 1,2-dimethyl butylene, 1,3-dimethyl butylene, 2,2-dimethyl butylene, 2,3-dimethyl butylene, 3,3-dimethyl butylene, 1-ethyl butylene, 2-ethyl butylene, 3-ethyl butylene, 1-n-propyl propylene, 2-n-propyl propylene, 1-iso-propyl propylene, 2-iso-propyl propylene, 1-methyl pentylene, 2-methyl pentylene, 3-methyl pentylene and 4-methyl pentylene. More preferably, said alkylene moiety has 1 to 3 carbon atoms, such as methylene, ethylene, n-propylene and iso-propylene. Most preferred is methylene.

The compounds of structural formula (I) are effective as melanocortin receptor modulators and are particularly effective as selective modulators of MC-4R. They are therefore useful for the treatment and/or prevention of disorders responsive to the activation and inactivation of MC-4R, such as cancer cachexia, muscle wasting, anorexia, anxiety, depression, obesity, diabetes, sexual dysfunction and other diseases with MC-4R involvement.

### Optical Isomers - Diastereomers - Geometric Isomers - Tautomers

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Some of the compounds described herein may exist as tautomers, such as keto-enol tautomers. The individual tautomers, as well as mixtures thereof, are encompassed within the compounds of structural formula (I).

Compounds of structural formula (I) may be separated into their individual diastereoisomers by, for example, fractional crystallization from a suitable solvent, for example, methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the structural formula (I) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, ptoluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

### Utility

Compounds of formula (I) are melanocortin receptor modulators and, as such, are useful in the treatment, control or prevention of diseases, disorders or conditions responsive to the activation or inactivation of one or more of the melanocortin receptors including, but not limited to, MC-1R, MC-2R, MC-3R, MC-4R or MC-5R. Such diseases, disorders or conditions include, but are not limited to, cancer cachexia, muscle wasting, anorexia, anxiety, depression, obesity (by reducing appetite, increasing metabolic rate, reducing fat intake or reducing carbohydrate craving), diabetes mellitus (by enhancing glucose tolerance, decreasing insulin resistance), hypertension, hyperlipidemia, osteoarthritis, cancer, gall bladder disease, sleep apnea, depression, anxiety, compulsion, neuroses, insomnia/sleep disorder, substance abuse, pain, male and female sexual dysfunction (including impotence, loss of libido and erectile dysfunction), fever, inflammation, immune-modulation, rheumatoid arthritis, skin tanning, acne and other skin disorders, neuroprotective and cognitive and memory enhancement including the treatment of Alzheimer's disease.

Some compounds encompassed by formula (I) show highly selective affinity for the melanocortin-4 receptor relative to MC-1R, MC-2R, MC-3R and MC-5R, which makes them especially useful in the prevention and treatment of cancer cachexia, muscle wasting, anorexia, anxiety, depression and obesity, as well as male and/or female sexual dysfunction, including erectile dysfunction. "Male sexual dysfunction" includes impotence, loss of libido and erectile dysfunction. "Female sexual dysfunction" can be seen as resulting from multiple components including dysfunction in desire, sexual arousal, sexual receptivity and orgasm.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia, generally, satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally, satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia, as well as other diseases or disorders for which compounds of formula (I) are useful, generally, satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, compounds of the present invention are given in a dose range of 0.001 milligram to about 100 milligram per kilogram of body weight, preferably as a single dose orally or as a nasal spray.

### Formulation

The compound of formula (I) is preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl, propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formula (I), the terms "A moiety", "B moiety" and "C moiety" are used below. This moiety concept is illustrated below:

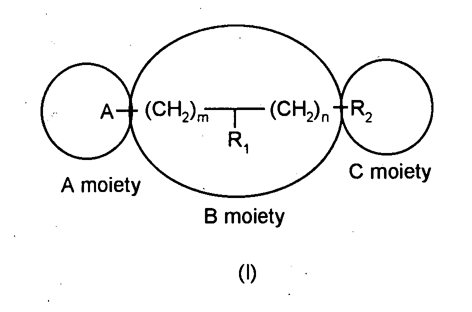

Preparation of the compounds of the present invention may be carried out via sequential or convergent synthetic routes. The "A" and "B moieties" of a compound of formula (I) are connected by reductive amination or nucleophilic substitution reactions. Those skilled in the art know various pathways and methods of connecting these two moieties using standard procedures. The skilled artisan will recognize that, in some embodiments, the "B" "C moieties" of a compound of formula (I) are connected via amide bonds. The skilled artisan can, therefore, readily envision numerous routes and methods of connecting these two moieties via standard peptide coupling reaction conditions.

The phrase "standard peptide coupling reaction conditions" means coupling a carboxylic acid with an amine using an acid activating agent such as EDC, dicyclohexylcarbodiimide, and benzotriazol-1-yloxytris(dimethylamino)-phosphonium hexafluorophosphate in a inert solvent such as DCM, in the presence of a catalyst such as HOBt. The uses of protective groups for amine and carboxylic acids to facilitate the desired reaction and minimize undesired reactions are well documented. Conditions required to remove protecting groups, which may be present, can be found in Greene, et al., Protective Groups in Organic Synthesis, John Wiley & Sons, Inc., New York, NY 1991.

Protecting groups like Z, Boc and Fmoc are used extensively in the synthesis and their removal conditions are well known to those skilled in the art. For example, removal of Z groups can he achieved by catalytic hydrogenation with hydrogen, in the presence of a noble metal or its oxide, such as palladium, on activated carbon in a protic solvent such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate, with a strong acid such as TFA or HCl or hydrogen chloride gas.

The compounds of Formula (I), when existing as a diastereomeric mixture, may be separated into diastereomeric pairs of enantiomers by fractional crystallization from a suitable solvent such as methanol, ethyl acetate or a mixture thereof. The pair of enantiomers, thus obtained, may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of Formula (I) of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The Examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The amine freebases corresponding to the isolated salts can be generated by neutralization with a suitable base, such as aqueous sodium hydrogencarbonate, sodium carbonate, sodium hydroxide and potassium hydroxide, and extraction of the liberated amine freebase into an organic solvent, followed by evaporation. The amine freebase isolated in this manner can be further converted into another pharmaceutically acceptable salt by dissolution in an organic solvent, followed by addition of the appropriate acid and subsequent evaporation, precipitation or crystallization. All temperatures are degrees Celsius. Mass spectra (MS) were measured by electron-spray ion-mass spectroscopy.

In the schemes, preparations and examples below, various reagent symbols and abbreviations have the following meanings:
- BINAP: 2,2'-bis(diphenylphosphino )-1,1'-binaphtyl
- Boc: t-butoxycarbonyl
- Bu: butyl
- Bz₂O₂: dibenzoylperoxide
- DCM: dichloromethane
- DIEA: diisopropylethylamine
- DMAP: 4-dimethylaminopyridine
- DME: dimethoxyethane
- DMF: N,N-dimethylformamide
- EDC: 1-(3-dimethylaminopropyl)-3-ethylcarbodiimide hydrochloride
- Et: ethyl
- EtOAc: ethyl acetate
- Fmoc: 9-fluorenylmethyl-carbamate
- HOAc: acetic acid
- HOAt: 1 -hydroxy-7-azabenzotriazole
- HOBt: 1-hydroxybenzotriazole
- h: hour(s)
- iPr: isopropyl
- NBS: N-bromosuccinimide
- NMM: N-methylmorpholine
- Me: methyl
- Ms: methanesulfonyl
- Pd₂(dba)₃: tris(dibenzylideneacetone) dipalladium(0)
- Phe: phenylalanine
- TEA: triethylamine
- TFA: trifluoroacetic acid
- Tf: trifluormethanesulfonyl
- THF: tetrahydrofuran
- Tic: 1,2,3,4-tetrahydroisoquinoline-3-carboxylic acid
- TLC: thin layer chromatography
- TMOF: trimethylorthoformate
- TMS: trimethylsilyl
- p-Ts: para-toluenesulfonyl
- Z: benzyloxycarbonyl

### Coupling of the three moieties:

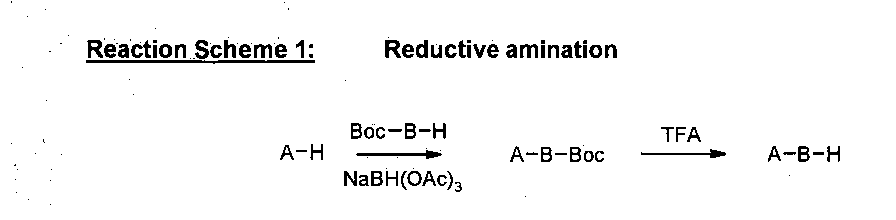

For coupling of H-A with amino aldehydes Boc-B-H, NaBH(OAc)₃, NaBH₄, or NaBH₃CN can be used.

Generally, the starting material of Boc-protected amine (Boc-A) can be deprotected in the presence of TFA/CH₂Cl₂, HCl/EtOAc, HCl/dioxane or HCl in MeOH/Et₂O with or without a cation scavenger, such as dimethyl sulfide (DMS), before being subjected to the coupling procedure.

A suitable solvent such as MeOH, EtOH or isopropanol, or a mixture of the above solvents, can be used for the coupling procedure, with or without addition of acetic acid.

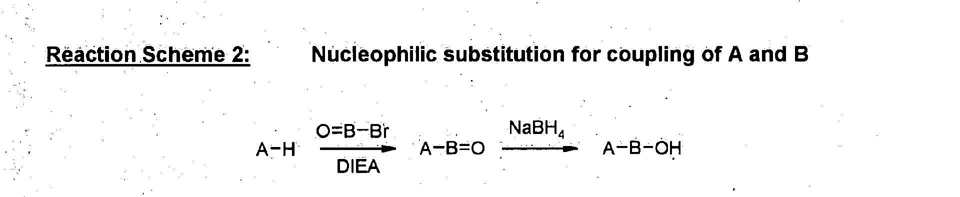

For coupling of H-A with phenacyl halides O=B-Br, DIEA, TEA, NMM, collidine or 2,6-lutidine, can be used as base.

Generally, the starting material of Boc-protected amine (Boc-A) can be deprotected in the presence of TFA/CH₂Cl₂, HCl/EtOAc, HCl/dioxane or HCl in MeOH/Et₂O, with or without a cation scavenger such as dimethyl sulfide (DMS), before being subjected to the coupling procedure.

A suitable solvent such as DCM, Et₂O, THF or DMF, or a mixture of the above solvents, can be used for the coupling procedure.

The ketones A-B=O can be reduced using NaBH₄, NaBH(OAc)₃ or NaBH₃CN in a suitable solvent, such as MeOH, EtOH or isopropanol, or a mixture of the above solvents.

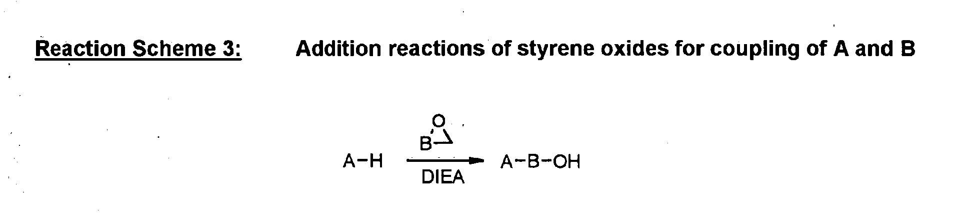

For coupling of H-A with styrene oxides (e.g. (R)-styrene oxide), DIEA, TEA, NMM, collidine or 2,6-lutidine can be used as base. Suitable solvents are DCM, DMF, DMSO, MeCN or THF and mixtures thereof.

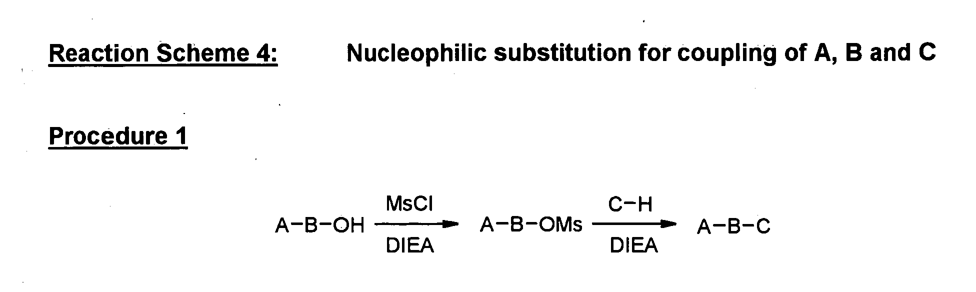

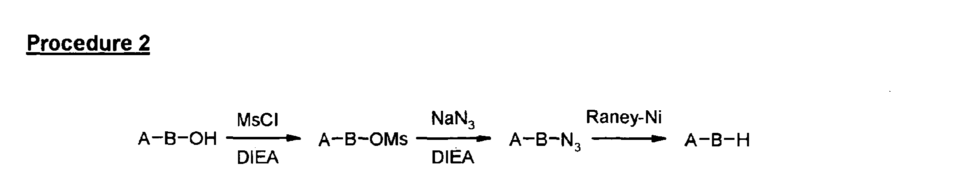

The OH function of benzylic alcohols A-B-OH can be transformed into a leaving group using MsCl, p-TsCl or Tf₂O in the presence of a suitable base like TEA, DIEA or NMM. Suitable solvents are DCM, THF, dioxane or pyridine, or a mixture thereof. When pyridine is used as a solvent, no additional base is required.

Intermediates A-B-OMs can directly be coupled with C-H (e g. 1-methyl-piperazine, Procedure 1) or they can be transformed into the corresponding azides with NaN₃ or TMSN₃, followed by subsequent reduction thereof (Procedure 2) yielding compounds A-B-H, which can be used for the peptide coupling described below.

The reaction of A-B-OH to A-B-C and A-B-N₃, respectively, can also be accomplished using Mitsunobu conditions.

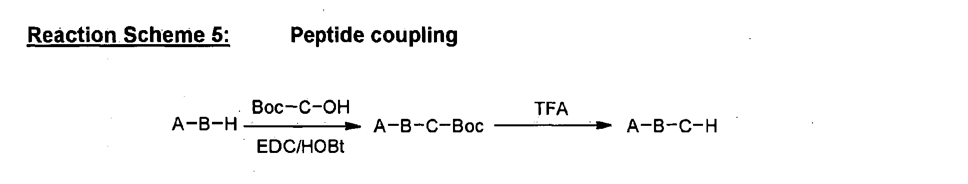

For coupling of H-BA with Boc-C-OH, EDC/HOAt, EDC/HOBt or DCC/HOBt can be used.

Generally, the starting material of Boc-protected amine (Boc-BA) can be deprotected in the presence of TFA/CH₂Cl₂, HCl/EtOAc, HCl/dioxane or HCl in MeOH/Et₂O, with or without a cation scavenger, such as dimethyl sulfide (DMS), before being subjected to the coupling procedure. It can be freebased before being subjected to the coupling procedure, or in some cases, used as the salt.

A suitable solvent such as CH₂Cl₂, DMF or THF, or a mixture of the above solvents, can be used for the coupling procedure. A suitable base includes TEA, DIEA, NMM, collidine or 2,6-lutidine.

A base may not be needed when EDC/HOBt is used.

Generally after the reaction is completed, the reaction mixture can be diluted with an appropriate organic solvent, such as EtOAc, CH₂Cl₂ or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

Protecting groups such as Boc, Z, Fmoc and CF₃CO can be deprotected in the presence of H₂/Pd-C, TFA/DCM, HCl/EtOAc, HCl/dioxane, HCl in MeOH/Et₂O, NH₃/MeOH or TBAF, with or without a cation scavenger such as thioanisole, ethane thiol or dimethyl sulfide (DMS). The deprotected amines can be used as the resulting salt or are freebased by dissolving in DCM and washing with aqueous bicarbonate or aqueous NaOH. The deprotected amines can also be freebased by ion exchange chromatography.

The "A", "B" and "C moieties" of the present invention, in general, may be prepared from commercially available starting materials via known chemical transformations.

### Reaction Schemes for Preparation of "A moiety"

The preparation of "A moiety" of the compound of formula (I) is illustrated in the reaction schemes below.

Some "A moieties" can be prepared as described in the corresponding literature:

4-Cyclohexyl-4-[1,2,4]triazol-1-ylmethyl-piperidine (WO0074679), 4-cyclohexyl-piperidine-4-carboxylic acid tert-butylamide (WO0170708) and 1,2-dihydro-1-methanesulfonylspiro-[3H-indole-3,4'-piperidine] (US005536716).

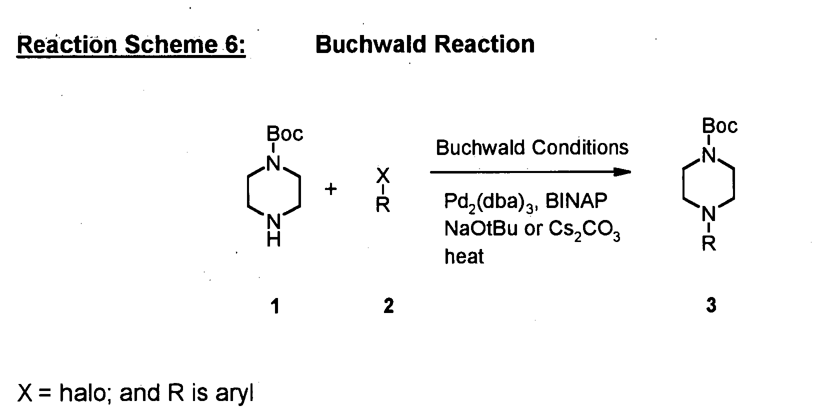

As shown in Reaction Scheme 6, the "A moiety" of the compounds of the present invention can be prepared by coupling halo-substituted aryl **2** (X-R) with 1-Boc-piperazine **1** in the presence of tri(dibenzylideneacetone) dipalladium (Pd₂(dba)₃), 2,2'-Bis(diphenylphosphino)-1,1'-binaphtyl (BINAP) and sodium-tert-butoxide (NaOtBu), or cesium carbonate (Cs₂CO₃), in an organic solvent such as toluene at a suitable temperature. More detailed examples of "A moiety" preparation are described below.

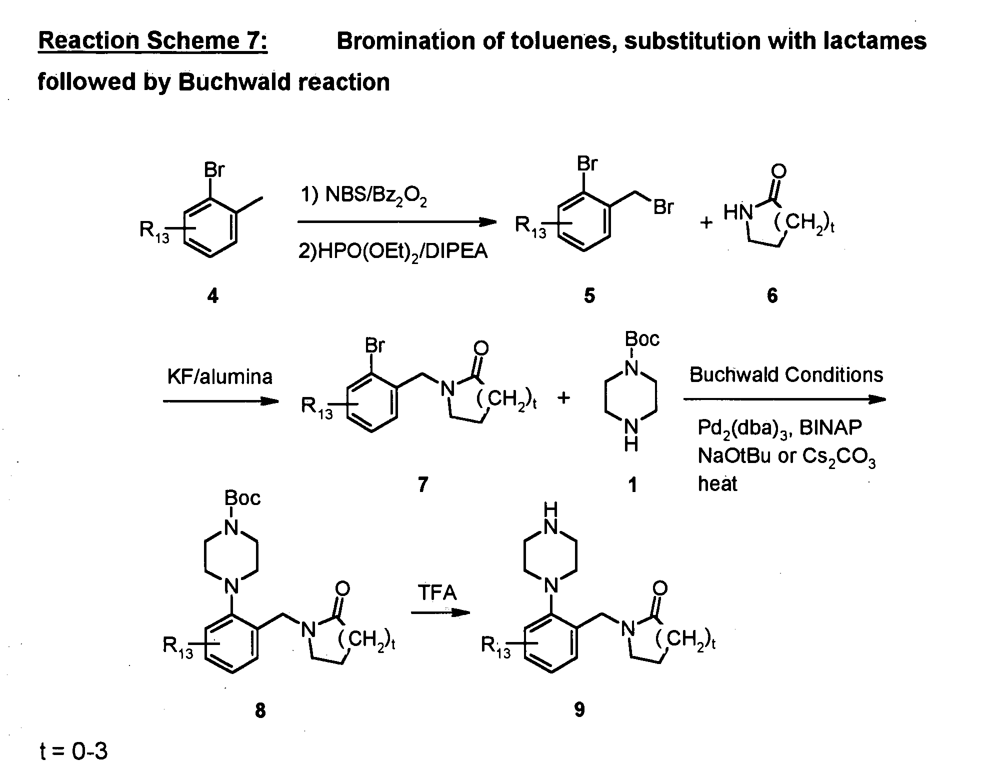

As shown in Reaction Scheme 7, the "A moiety" of the compounds of the present invention can be prepared by reacting various methyl benzenes **4** with NBS in the presence of a radical starter such as Bz₂O₂, followed by reaction with diethyl phosphite, in the presence of a base such as DIPEA, to give benzylbromides **5**, which can then used to alkylate lactames, like **6**, in the presence of a appropriate base such as KF/alumina. The substituted bromobenzenes can then be subjected to Buchwald conditions, followed by deprotection using an appropriate reactant such as TFA.

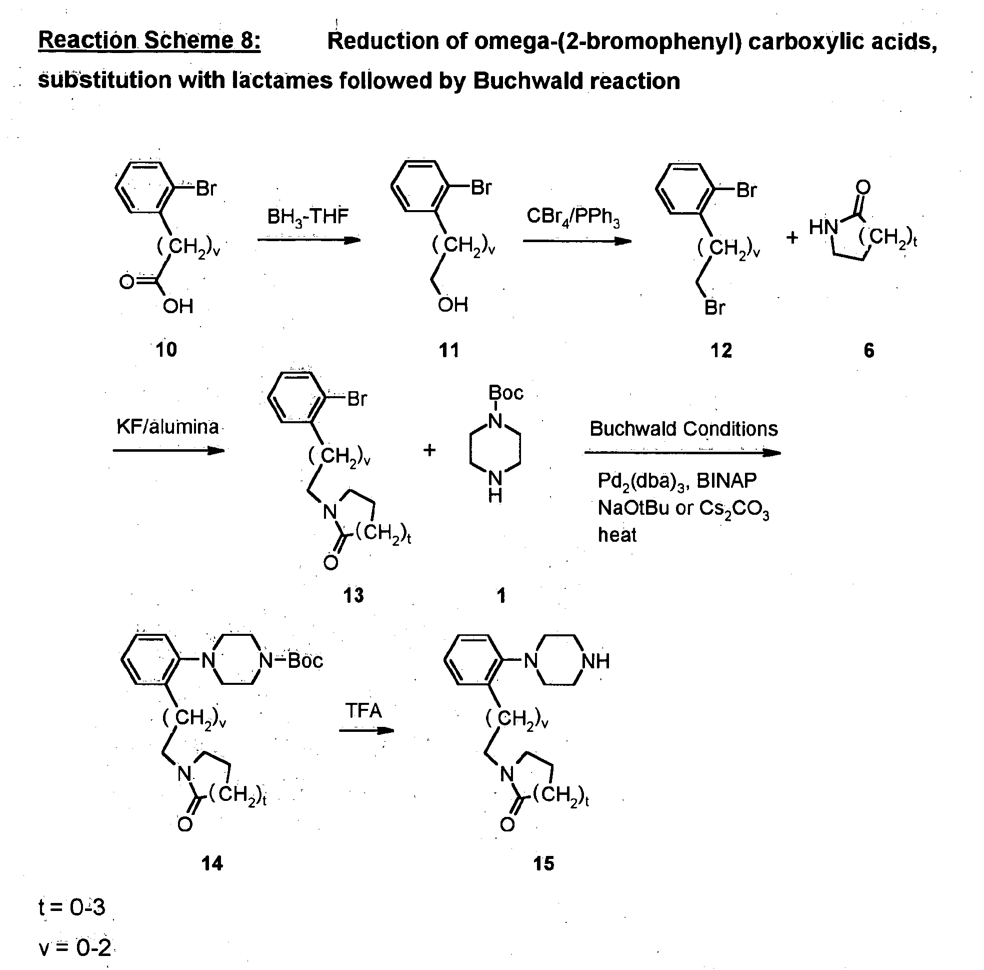

As shown in Reaction Scheme 8, carboxylic acids **10** can be reduced to the corresponding alcohols **11**, using an appropriate reagent such as BH₃-THF, which are subsequently transferred to the corresponding alkyl bromides **12** with reagents such as CBr₄ and PPh₃. The alkyl bromides can then be used to alkylate lactames, like **6**, in the presence of an appropriate base such as KF/alumina. The substituted bromobenzenes can then be subjected to Buchwald conditions, followed by deprotection, using an appropriate reactant such as TFA.

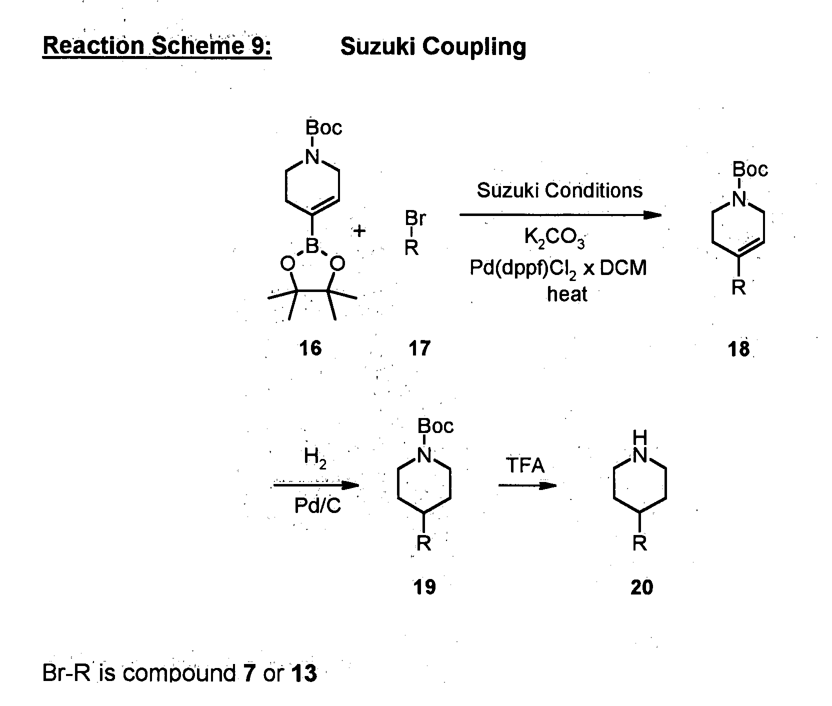

As shown in Reaction Scheme 9, 1-(2(H)-pyridine-carboxylic acid-3,6-dihydro-4-(4,4,5,5-tetramethyl-1,3,2-dioxaborolan-2-yl)-1,1-dimethyl ethyl ester **16** *(Tetrahedron Lett.* 2000, 41, 3705-3708) can be reacted with halo aryl compounds, such as **7** or **13**, in the presence of a base such as K₂CO₃ and a catalyst such as dichloro(1,1'-bis(diphenylphosphino)-ferrocene)palladium(II) DCM adduct, in an organic solvent such as DMF, or toluene, at a suitable temperature. The tetrahydropyridines can be hydrogenated in the presence of a catalyst, such as Pd/C, to yield the protected piperidines **19**, which can subsequently be deprotected with a reagent, such as TFA, to yield piperidines **20**.

### Reaction Schemes for Preparation of "B moiety"

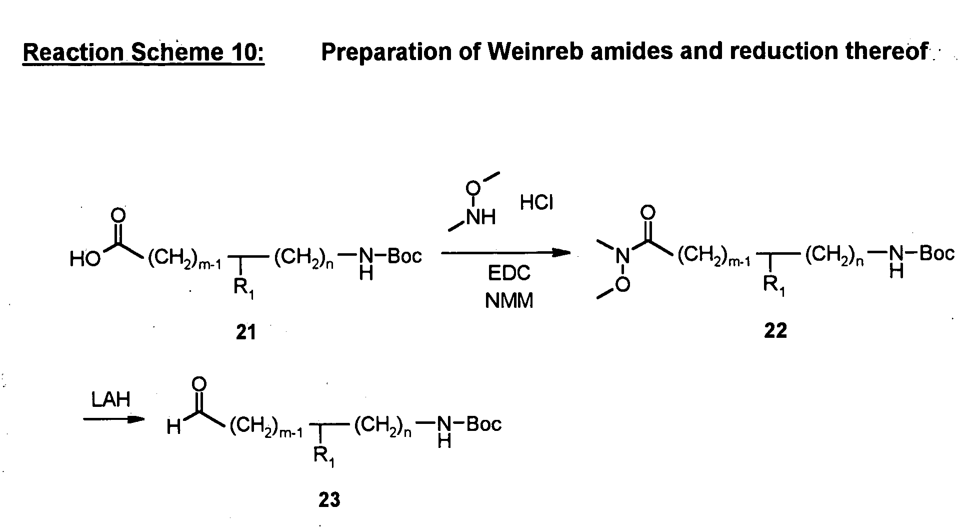

As shown in Reaction Scheme 10, amino acids **21** can be converted into the corresponding Weinreb amides using standard peptide coupling conditions such as EDC/NMM, in an appropriate solvent such as DCM (analog *Synth. Commun.* 1982, 676). Reduction of the Weinreb amides **22**, to the aldehydes **23**, can be performed with reagents like LAH, in an appropriate solvent such as diethyl ether *(Chirality* 2000, 12, 2).

### Reaction Schemes for Preparation of "C moiety"

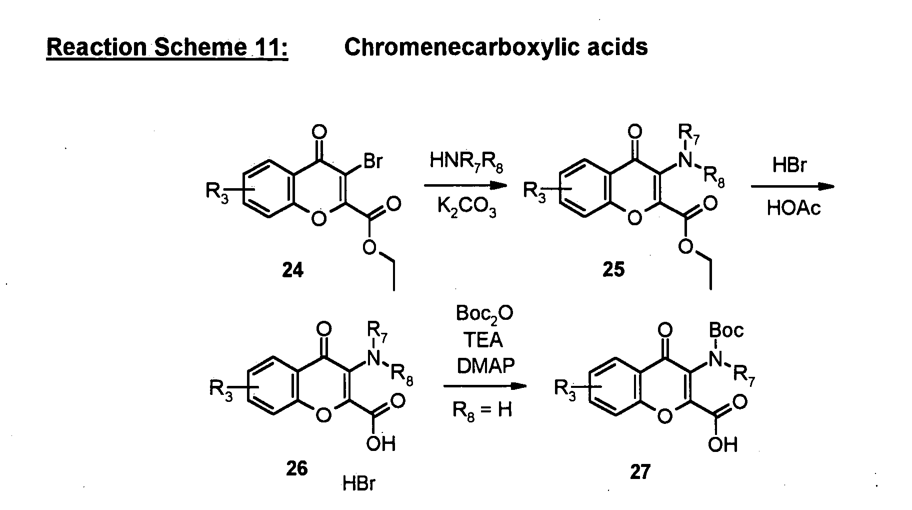

As shown in Reaction Scheme 11, ethyl 3-bromo-4-oxochromene-2-carboxylate **24** (J. *Chem. Soc. Perkin Trans.* /1986, 1643-1649) can be reacted with amines, with or without a base such as K₂CO₃, in an appropriate solvent such as MeCN, to form products **25**, which are subsequently treated with a reagent, such as HBr/HOAc, to form carboxylic acids **26**. When R₈ is hydrogen, the free-amine can be protected with a reagent, such as Boc₂O, in the presence of TEA and DMAP, in an appropriate solvent.

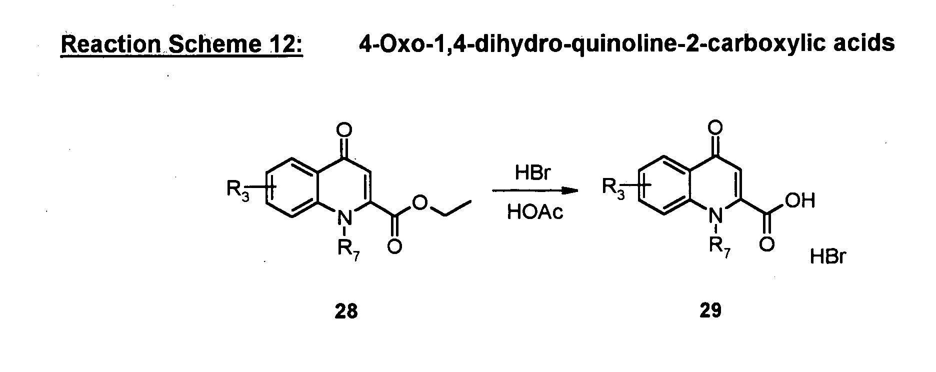

As shown in Reaction Scheme 12, ethyl 4-oxo-1,4-dihydro-quinoline-2-carboxylates **28** *(Bioorg. Med. Chem. Lett.* 2000, 10, 1487-1490) can be converted into the corresponding acids **29** by an appropriate reactant, such as HBr/HOAc.

The following describes the detailed examples of the invention.

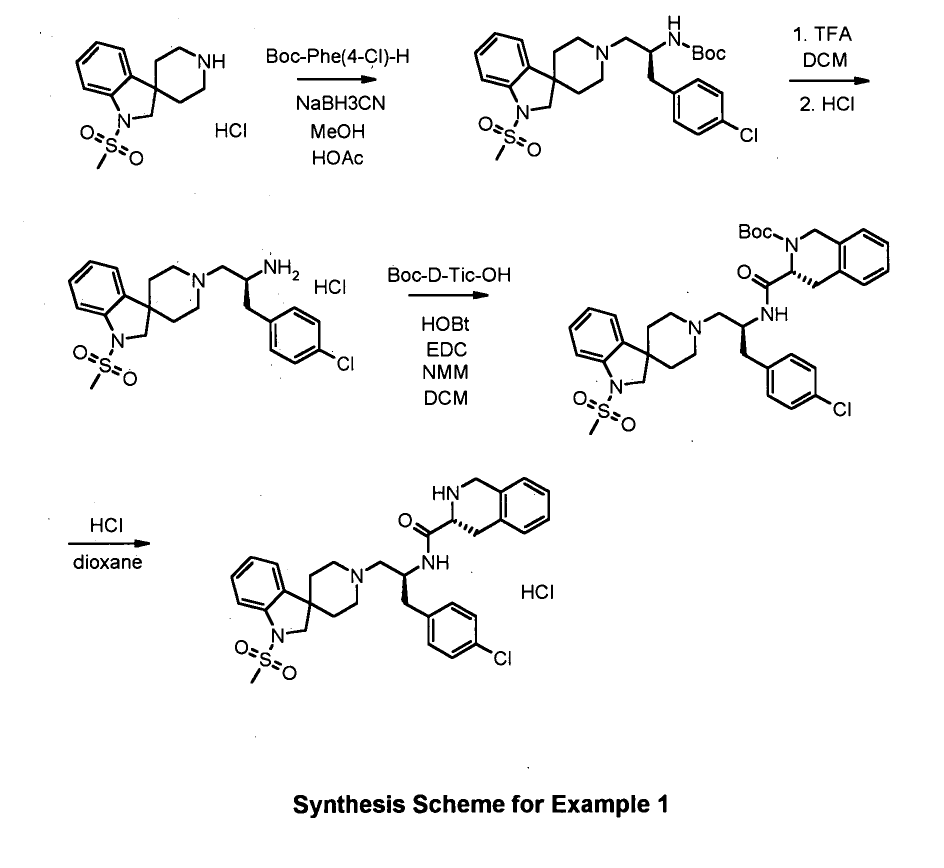

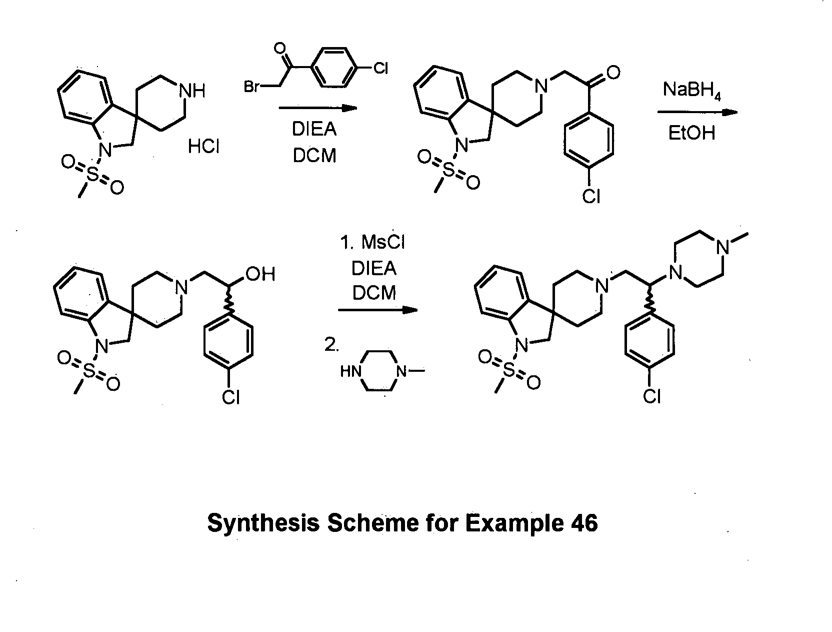

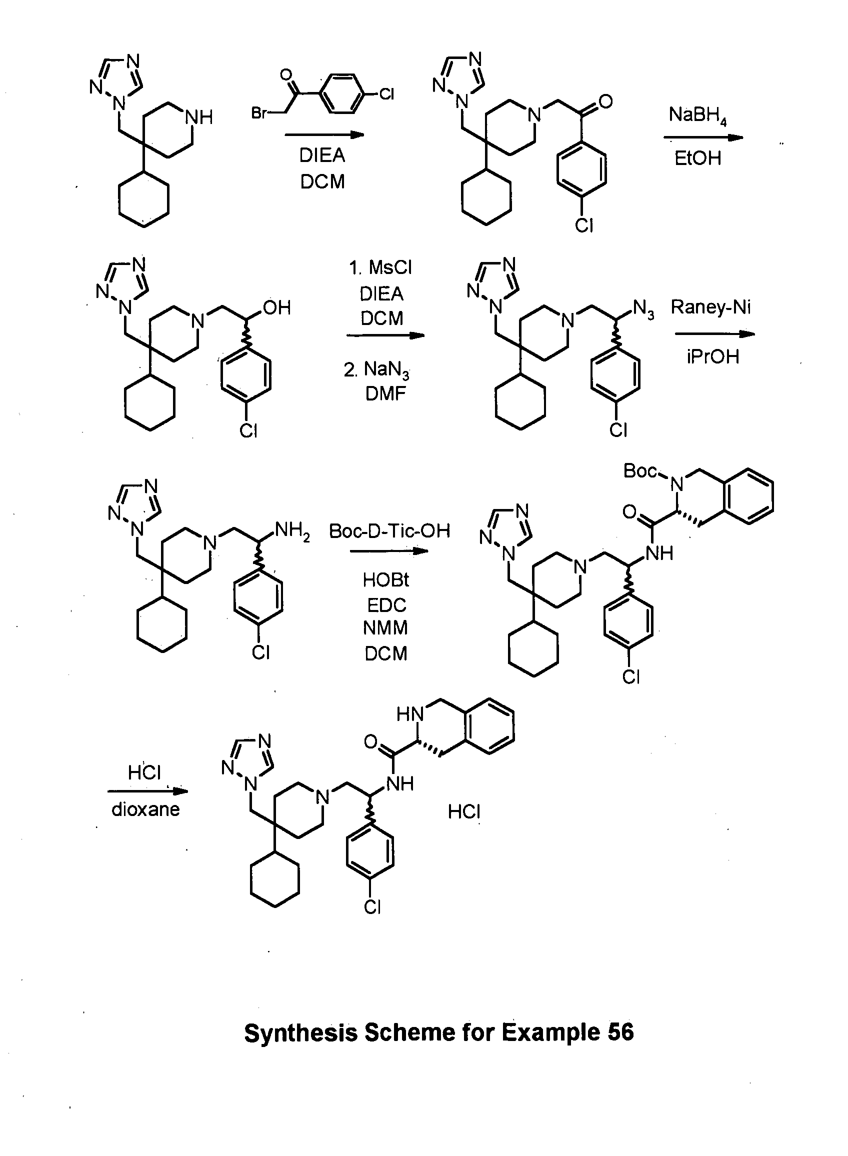

The following examples are provided to illustrate the invention and are not limiting the scope of the invention in any manner.

### Example 1:

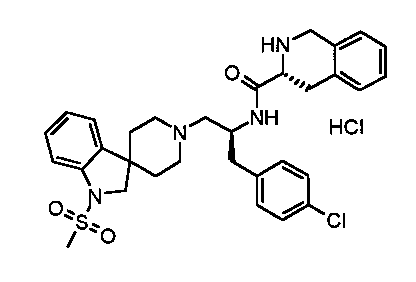

To Boc-protected intermediate 1c) (31 mg) was added hydrogen chloride, 4.0 M sol. in 1,4-dioxane (10 ml) and the solution was stirred for 90 min at room temperature. The solvent was removed under reduced pressure. The residue was dissolved in DCM and treated with diethyl ether. The precipitate was filtered off to yield the title compound as a solid.

The required intermediates can be synthesized in the following way:

### Intermediate 1a):

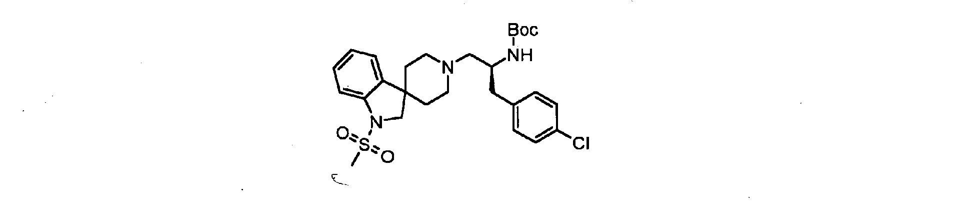

To a solution of 1,2-dihydro-1 -methanesulfonylspiro-[3H-indole-3,4'-piperidine] hydrochloride (152 mg) in methanol (3.2 ml) and acetic acid (0.8 ml) was added Boc-L-4-chlorophenylalaninal (142 mg) and stirred for 90 min. After cooling to 0°C, sodium cyanoborohydride (47 mg) was added in small portions. The reaction mixture was stirred for 1 h and partitioned between sat. NaHCO₃ and DCM. The aqueous phase was extracted two times with DCM. The combined organic were dried over Na₂SO₄ and concentrated in vacuo. Purification by column chromatography yielded the title compound.

### Intermediate 1b):

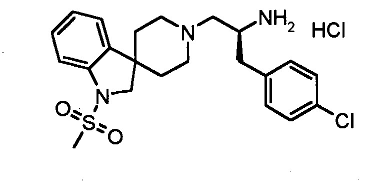

To the Boc-protected amine from 1a) (132 mg) in DCM (5 ml) was added TFA (1 ml) and stirred at room temperature for 90 min. Additional TFA (1 ml) was added and stirred for 10 min. The reaction mixture was diluted with DCM (10 ml) and carefully basified by pouring it into 10% aqueous sodium carbonate solution (20 ml). The organic layer was separated and the aqueous layer was further extracted three times with DCM. The combined organics were washed with water and brine, dried over Na₂SO₄ and concentrated to give a white solid.

For prolonged storage, the free-base was converted into the corresponding hydrochloride. The free-base was dissolved in DCM (5 ml) and app. 1 M HCl in ether (10 ml) was added. The precipitate was filtered and the residue was washed three times with ether and dried under reduced pressure to yield the desired compound.

### Intermediate 1c):

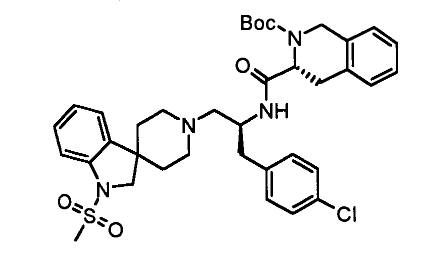

To (R)-Boc-1,2,3,4-tetrahydroisoquinoline-3-carboxylic acid (24 mg) in DCM (2 ml) was added intermediate 1b) (38 mg), N-methylmorpholine (14 µl) and HOBt (14 mg) and then it was stirred for 20 min. EDC (23 mg) was added and stirring continued for 1 h. An additional amount of N-methylmorpholine (8 µl) was added and stirred overnight. The reaction mixture was poured into water (5 ml) and the organic phase was separated The aqueous phase was extracted two times with DCM. The combined organic phases were washed with 0.5 N HCl and saturated sodium bicarbonate solution, dried over Na₂SO₄ and concentrated to yield the product which was purified by column chromatography.

The following examples can be prepared in a similar way:

### Example 2:

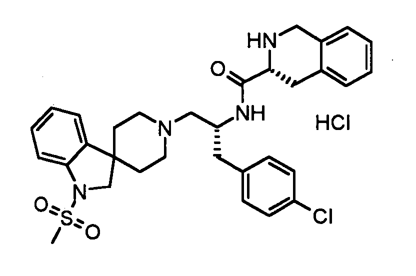

### Example 3:

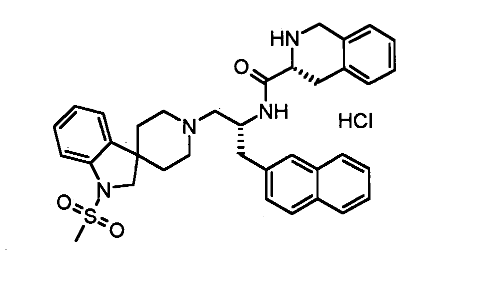

### Example 4:

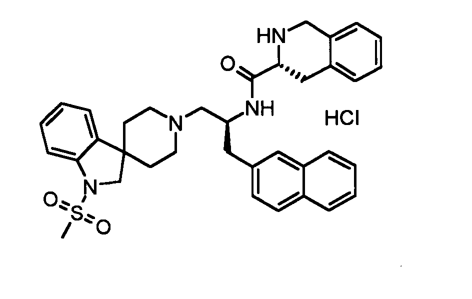

### Example 5:

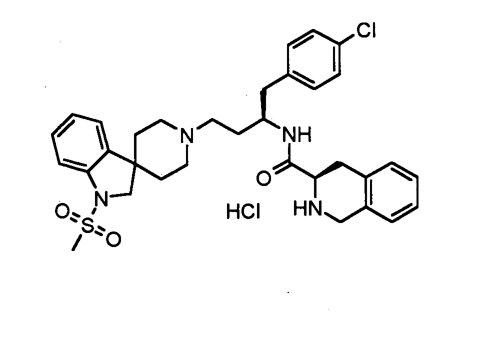

### Example 6:

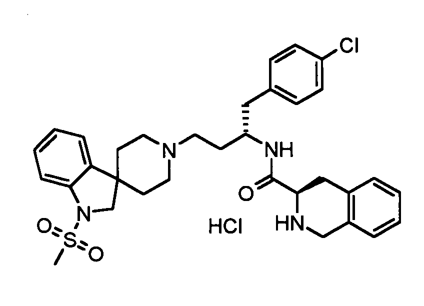

### Example 7:

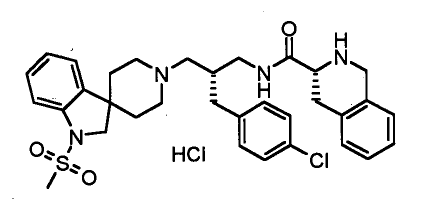

### Example 8:

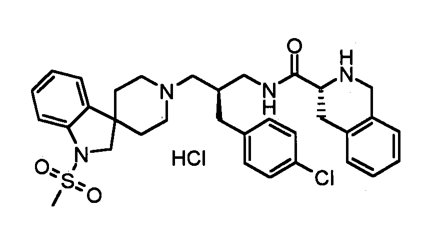

### Example 9:

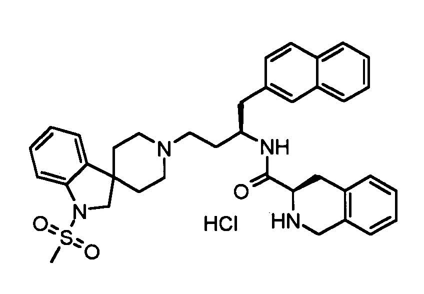

### Example 10:

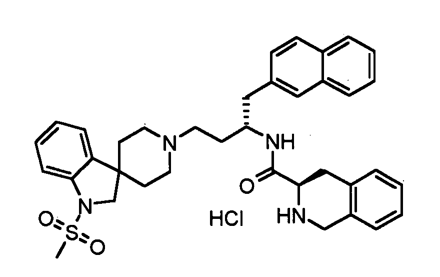

### Example 11:

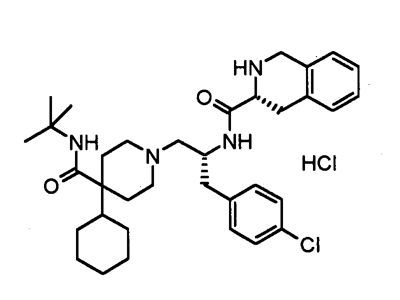

### Example 12:

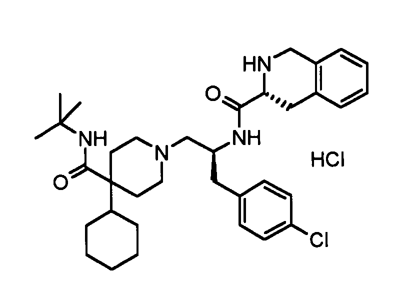

### Example 13:

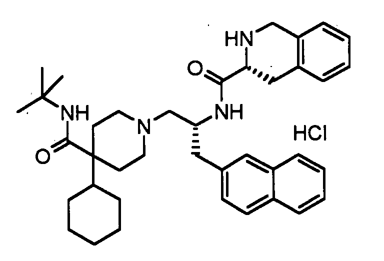

### Example 14:

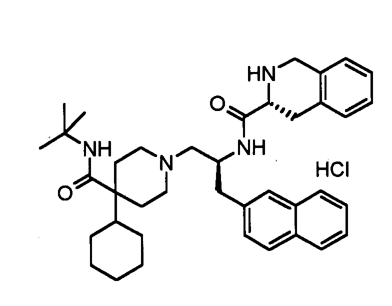

### Example 15:

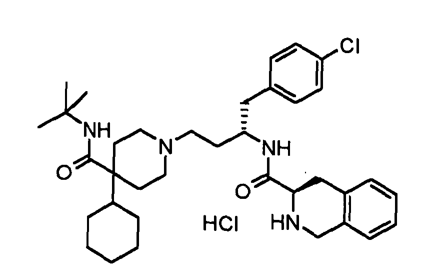

### Example 16:

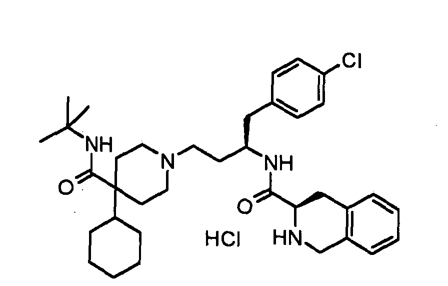

### Example 17:

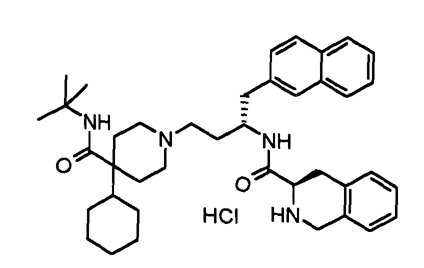

### Example 18:

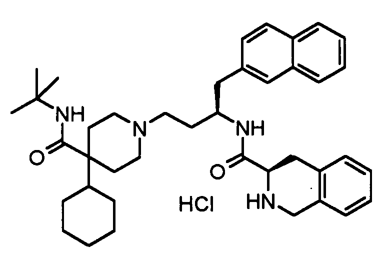

### Example 19:

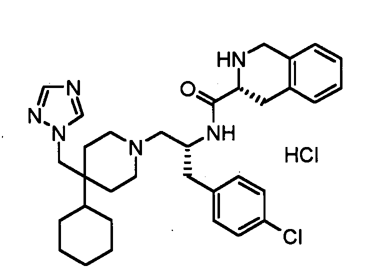

### Example 20:

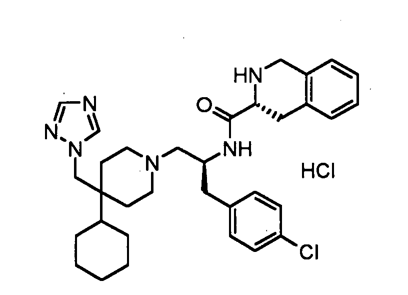

### Example 21:

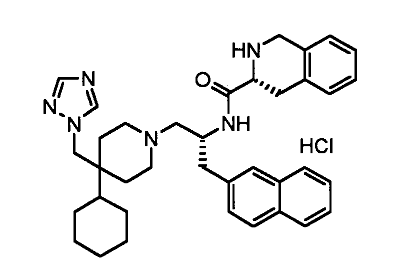

### Example 22:

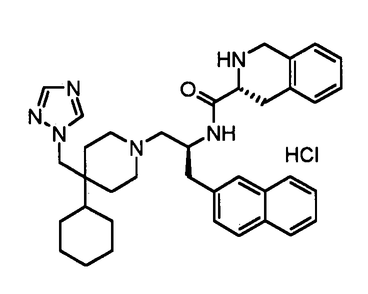

### Example 23:

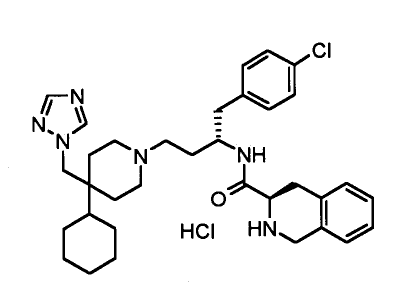

### Example 24:

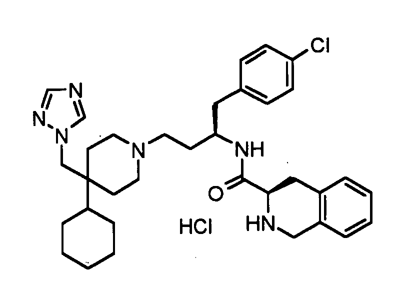

### Example 25:

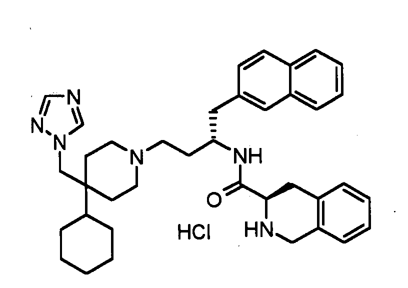

### Example 26:

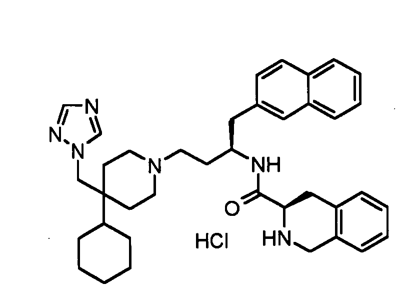

### Example 27:

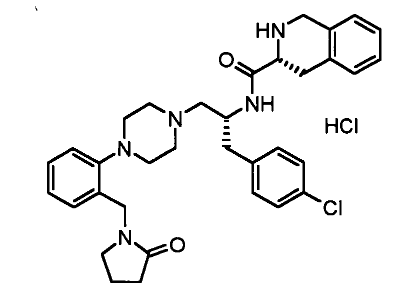

### Example 28:

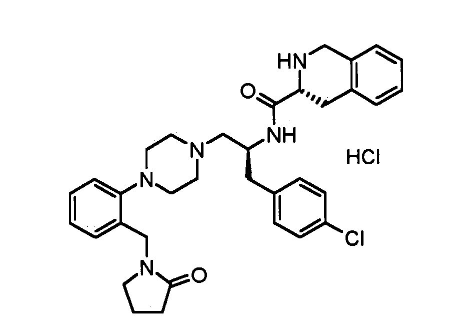

### Example 29:

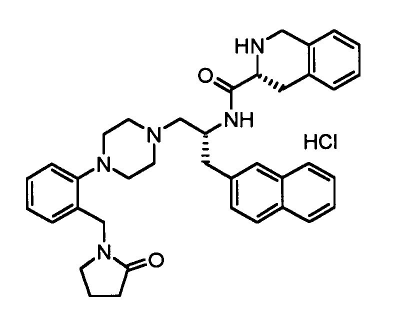

### Example 30:

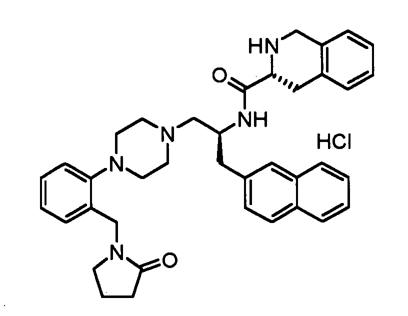

### Example 31:

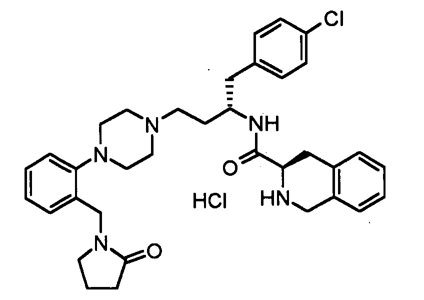

### Example 32:

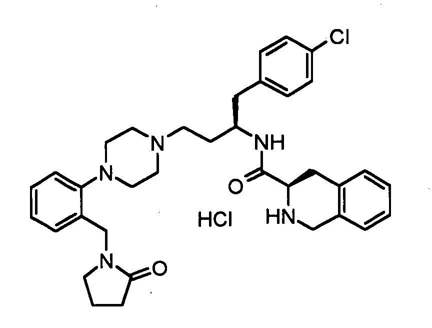

### Example 33:

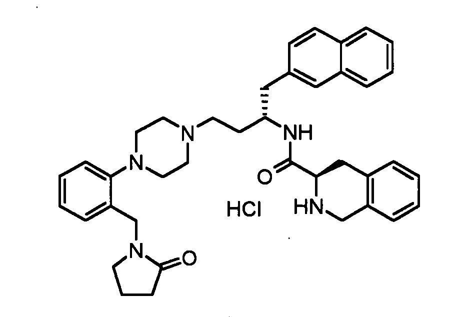

### Example 34:

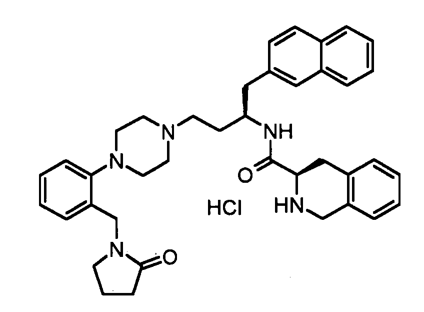

### Example 35:

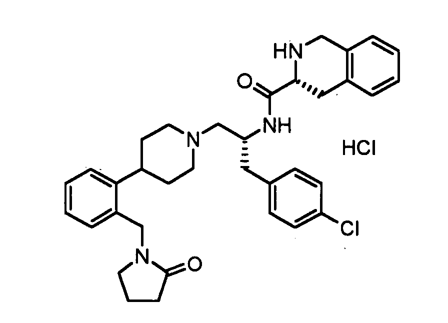

### Example 36:

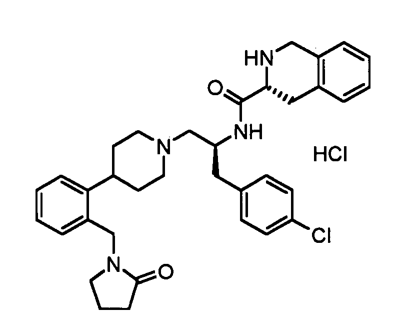

### Example 37:

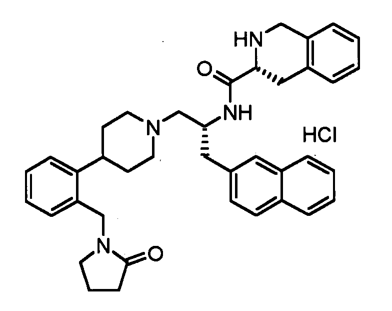

### Example 38:

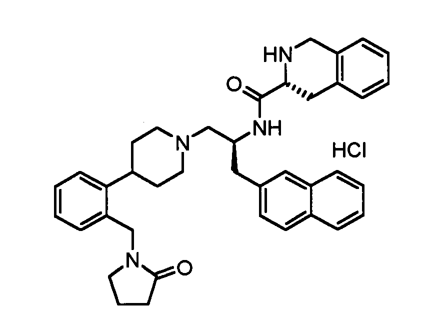

### Example 39:

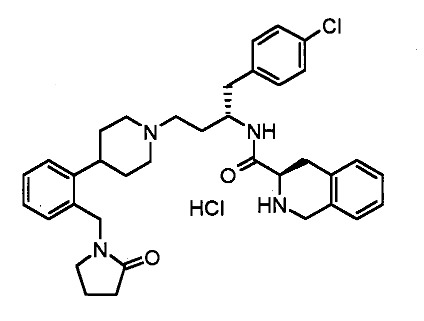

### Example 40:

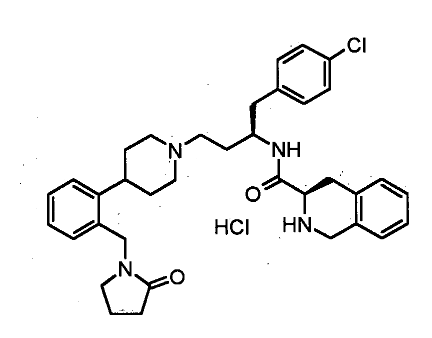

### Example 41:

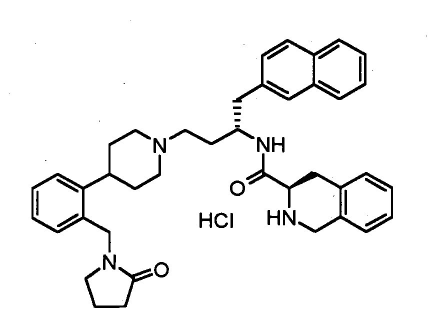

### Example 42:

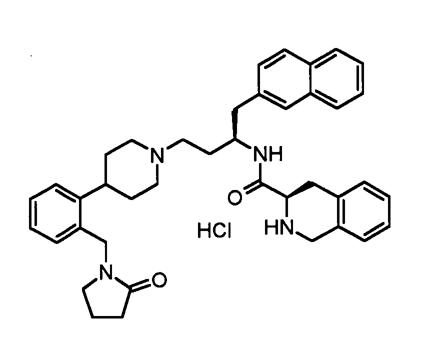

### Example 43:

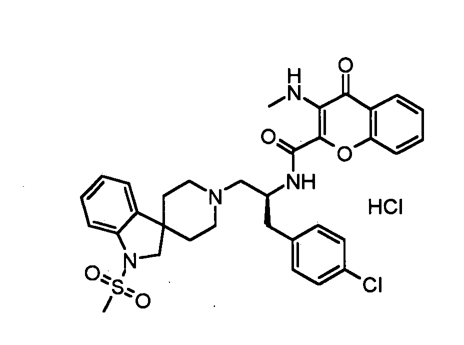

### Example 44:

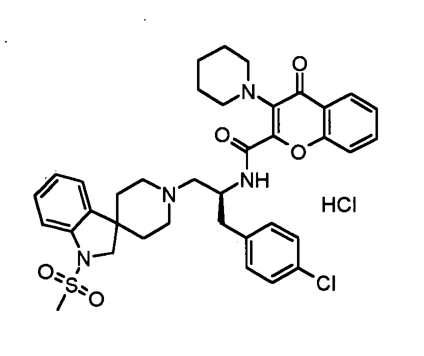

### Example 45:

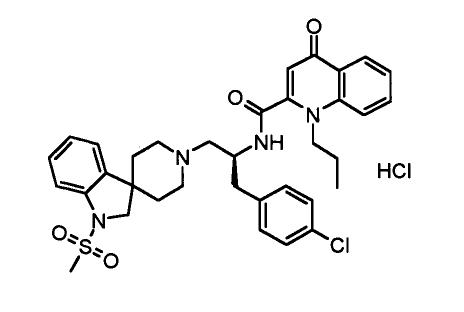

### Example 46:

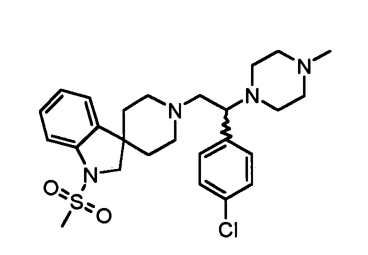

To a solution of intermediate 46c) (25 mg) in DCM was added N-methyl-piperazine (18 µl) and it was stirred overnight. The reaction mixture was diluted with DCM and extracted with water and brine. The organic layer was dried over Na₂SO₄ and concentrated to yield the title compound which was purified by column chromatography.

The required intermediates can be synthesized in the following way:

### Intermediate 46a):

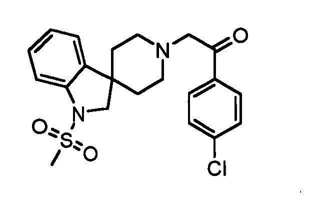

To a solution of 1,2-dihydro-1-methanesulfonylspiro-[3H-indole-3,4'-piperidine] hydrochloride (152 mg) and DIEA (258 µl) in DCM (5 ml) was added p-chlorophenacyl bromide (117 mg) and it was stirred overnight. The reaction mixture was diluted with DCM and then poured into water. The aqueous phase was extracted twice with DCM. The combined organic layers were washed with 1 M HCl and sat. NaHCO₃ and dried over Na₂SO₄. The title compound was obtained after evaporation of the solvent.

### Intermediate 46b):

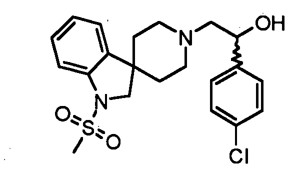

A stirred solution of intermediate 46a) (168 mg) in ethanol (6 ml) was treated at 30 - 40°C with NaBH₄ (16 mg). The mixture was stirred for 1 h without heating and then for 1 h at 50°C. After cooling the volatiles were removed under reduced pressure, the residue was diluted with water and extracted three times with DCM. The combined extracts were washed with water and bnne, dried over Na₂SO₄ and the solvent was evaporated to yield the title compound.

### Intermediate 46c):

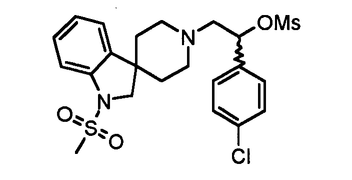

To a solution of intermediate 46b) (127 mg) in DCM (1 ml) was added TEA (84 µl). The reaction mixture was cooled to 0°C and methanesulfonyl chloride (47 µl) was added. After the reaction mixture was stirred for 90 min, volatiles were removed in vacuo and the residue was partitioned between water and EtOAc. The aqueous phase was extracted twice with EtOAc and the combined organic layers were washed with brine, dried over Na₂SO₄ and concentrated to afford the title compound.

The following examples can be prepared in a similar way:

### Example 47:

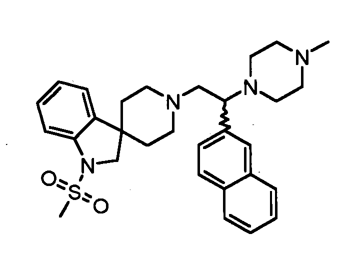

### Example 48:

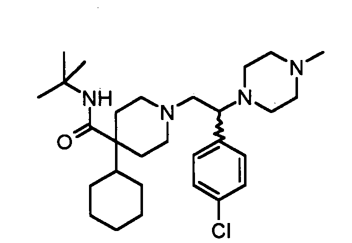

### Example 49:

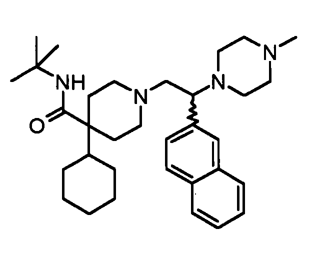

### Example 50:

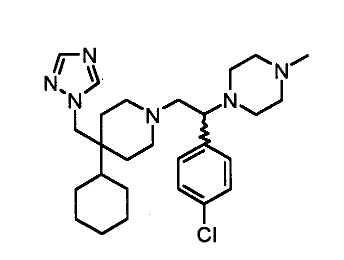

### Example 51:

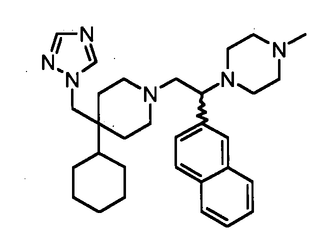

### Example 52:

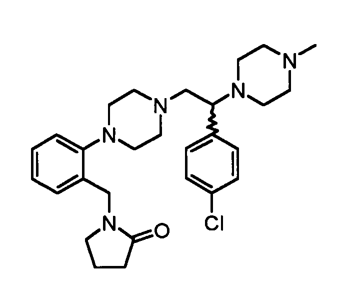

### Example 53:

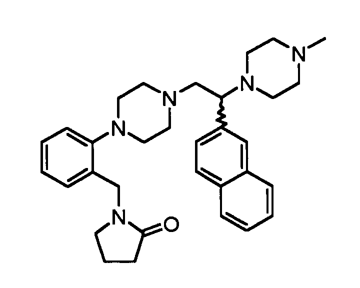

### Example 54:

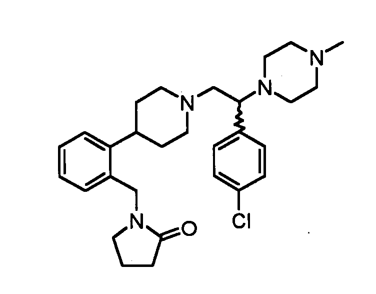

### Example 55:

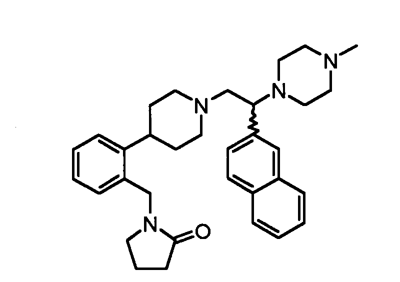

### Example 56:

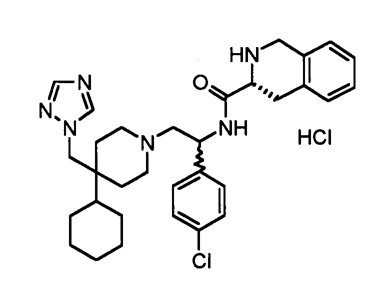

To Boc-protected intermediate 56f) (29 mg) was added hydrogen chloride, 4.0 M sol. in 1,4-dioxane (10 ml) and the solution was stirred for 90 min at room temperature. The solvent was removed under reduced pressure. The residue was dissolved in DCM and treated with diethyl ether. The precipitate was filtered off to yield the title compound as a solid.

The required intermediates can be synthesized in the following way:

### Intermediate 56a):

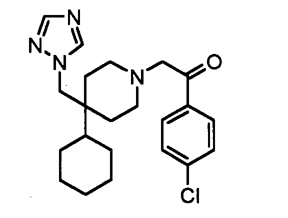

To a solution of 4-cyclohexyl-4-[1,2,4]triazol-1-ylmethyl-piperidine hydrochloride (143 mg) and DIEA (258 µl) in DCM (5 ml) was added p-chlorophenacyl bromide (117 mg) and stirred overnight. The reaction mixture was diluted with DCM and poured into water. The aqueous phase was extracted twice with DCM. The combined organic layers were washed with 1 M HCl and sat NaHCO₃, and dried over Na₂SO₄. The title compound was obtained after evaporation of the solvent.

### Intermediate 56b):

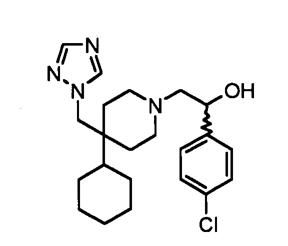

A stirred solution of intermediate 56a) (161 mg) in ethanol (6 ml) was treated at 30 - 40°C with NaBH₄ (16 mg). The mixture was stirred for 1 h without heating and then for 1 h at 50°C. After cooling the volatiles were removed under reduced pressure, the residue was diluted with water and extracted three times with DCM. The combined extracts were washed with water and brine, dried over Na₂SO₄ and the solvent was evaporated to yield the title compound.

### Intermediate 56c):

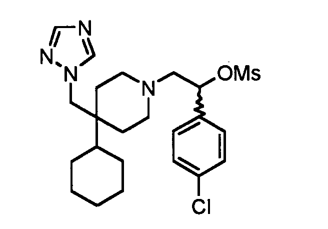

To a solution of intermediate 56b) (121 mg) in DCM (1 ml) was added TEA (84 µl). The reaction mixture was cooled to 0°C and methanesulfonyl chloride (47 µl) was added. After the reaction mixture was stirred for 90 min, volatiles were removed in vacuo and the residue was portioned between water and EtOAc. The aqueous phase was extracted twice with EtOAc and the combined organic layers were washed with brine, dried over Na₂SO₄ and concentrated to afford the title compound.

### Intermediate 56d):

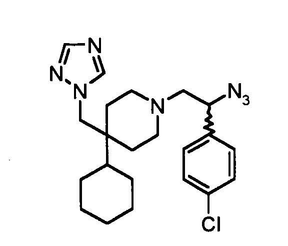

To a solution of intermediate 56c) (96 mg) in DMF (500 µl) was added sodium azide (26 mg). The reaction mixture was stirred for 48 h and then poured on water. The aqueous phase was extracted three times with EtOAc. The combined organic layers were washed with water and brine, and dried over Na₂SO₄. After evaporation of the solvent the desired compound was obtained.

### Intermediate 56e):

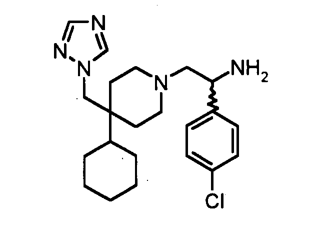

To a solution of intermediate 56d) (43 mg) in isopropanol (2 ml) was added Raney nickel (25 mg). The reaction was flushed with argon followed by hydrogen. The azide was hydrogenated at 1 atm hydrogen pressure overnight. The reaction mixture was filtered over Celite and the residue was washed with isopropanol twice. The combined filtrates were concentrated to afford the title compound.

### Intermediate 56f):

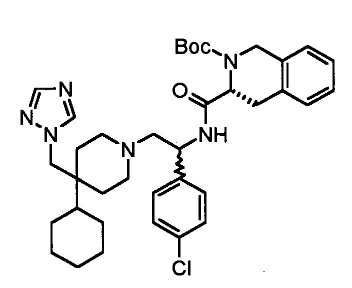

To (R)-Boc-1,2,3,4-tetrahydroisoquinoline-3-carboxylic acid (24 mg) in DCM (2 ml) was added intermediate 56e) (33 mg), N-methylmorpholine (14 µl), HOBt (14 mg) an it was stirred for 20 min. EDC (23 mg) was added and stirring continued for 1 h. An additional amount of N-methylmorpholine (8 µl) was added and then it was stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated to yield the product which was purified by column chromatography.

The following examples can be prepared in a similar way:

### Example 57:

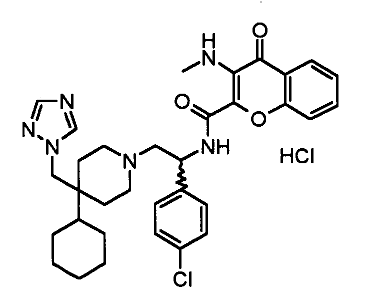

### Example 58:

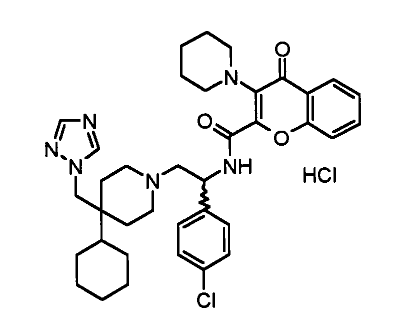

### Example 59:

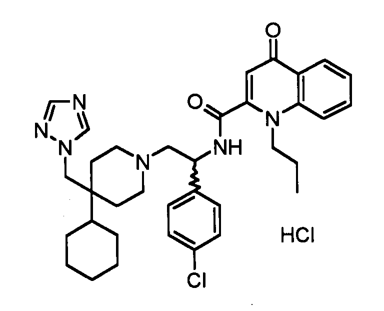

### Preparation of the carboxylic acids

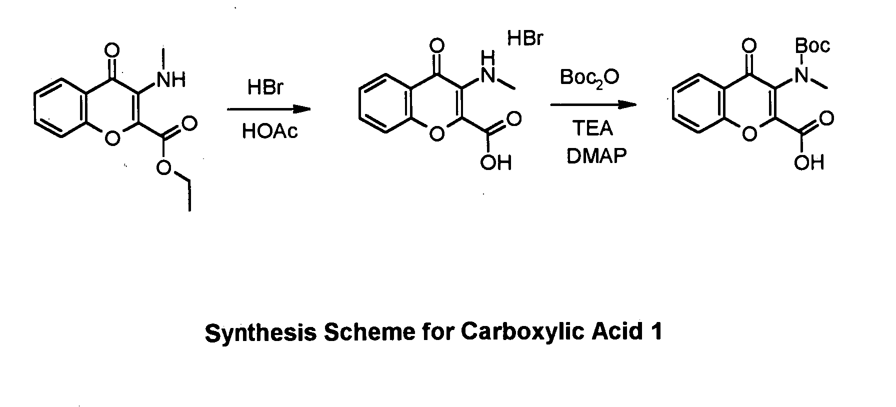

### Carboxylic Acid 1:

To a solution of intermediate CA1a) (0.4 g) in THF (10 ml) was added TEA (670 µl), DMAP (20 mg) and Boc₂O (384 mg). The reaction mixture was stirred overnight. The solvent was removed under reduced pressure and the residue was dissolved in ethyl acetate. The solution was extracted with water, 1 M HCl and sat. NaHCO₃, and dried over Na₂SO₄. The solvent was removed under reduced pressure to yield the title compound.

### Intermediate CA1a):

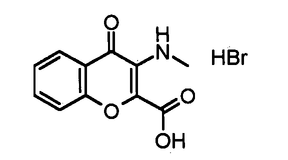

Ethyl 3-methylamino-4-oxochromene-2-carboxylate (0.8 g) was hydrolyzed by heating with hydrobromic acid (4 ml) and acetic acid (3 ml) for 4 h to give the desired compound after evaporation of the solvent.

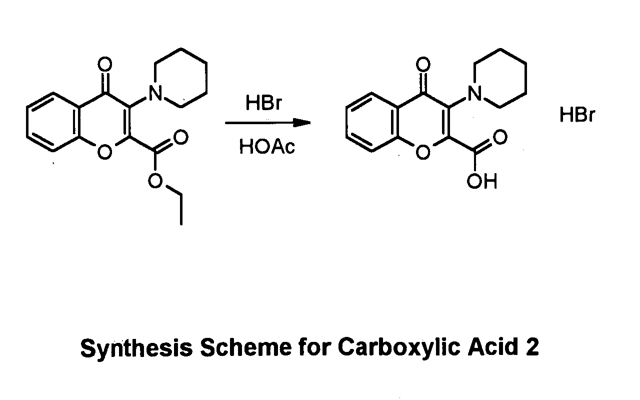

### Carboxylic Acid 2:

Ethyl 3-piperidino-4-oxochromene-2-carboxylate (0.8 g) was hydrolyzed by heating with hydrobromic acid (4 ml) and acetic acid (3 ml) for 4 h to give the desired compound after evaporation of the solvent.

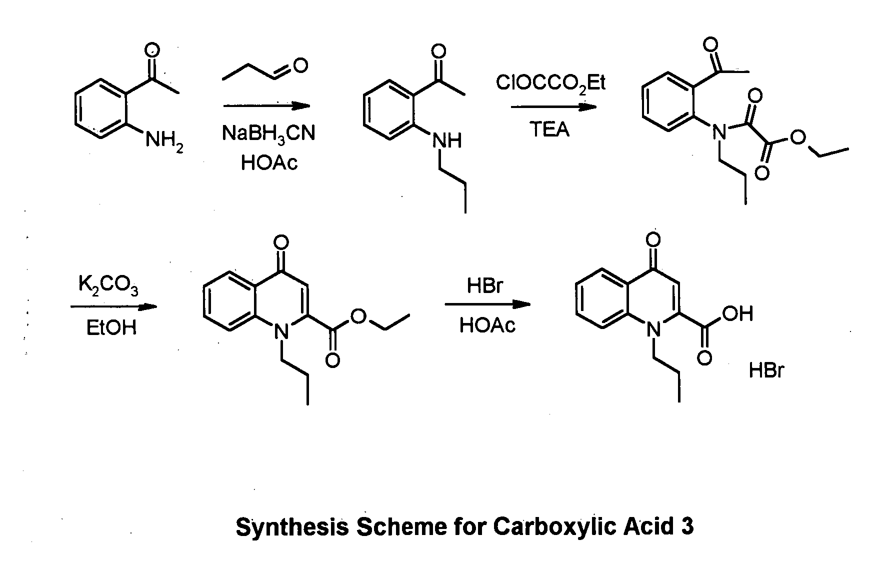

### Carboxylic Acid 3:

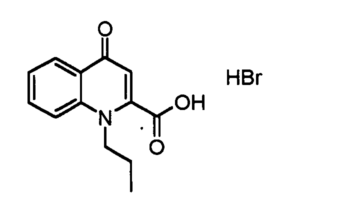

Intermediate CA3c) (0.1 g) was hydrolyzed by heating with hydrobromic acid (2 ml) and acetic acid (1.5 ml) for 3 h to give the desired compound after evaporation of the solvent.

### Intermediate CA3a):

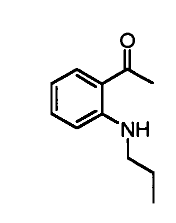

To a solution of 2'-aminoacetophenone (709 mg) in methanol (10 ml) was added propionaldehyde (580 µl) and TMOF (482 µl) and the solution was stirred overnight. The volatiles were removed under reduced pressure and the residue was redissolved in methanol (10 ml). Acetic acid (115 µl) and sodium cyanoborohydride (126 mg) were added and the reaction was stirred overnight. After basification with 1 M NaOH, the volatiles were removed under reduced pressure. The residue was dissolved in ethyl acetate and washed with water and brine. The organic phase was dried over Na₂SO₄ and the solvent was removed under reduced pressure to yield the title compound.

### Intermediate CA3b):

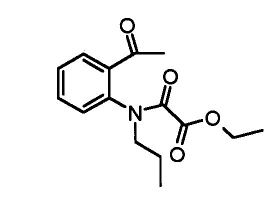

To a solution of intermediate CA3a) (681 mg) in dry THF (10 ml) was added TEA (670 µl) and the mixture was cooled to 0°C. At this temperature, ethyl oxalyl chloride (470 µl) was added dropwise. The reaction mixture was stirred for 4 h at room temperature. The volatiles were removed under reduced pressure and the residue was dissolved in ethyl acetate. The solution was washed with water, sat. NaHCO₃ and brine, and dried over Na₂SO₄ to yield the desired compound.

### Intermediate CA3c):

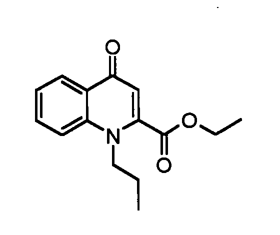

Intermediate CA3b) (555 mg) was dissolved in ethanol (10 ml). K₂CO₃ (276 mg) was added and the reaction mixture was stirred overnight. The reaction mixture was filtrated and the solvent was removed to yield the title compound.

### BIOLOGICAL ASSAYS

### A. Binding Assay

A membrane binding assay is used to identify competitive inhibitors of fluorescence labeled NDP-alpha-MSH binding to HEK293 cell membrane preparations, expressing human melanocortin receptors.

The test compound, or unlabeled NDP-alpha-MSH, is dispensed at varying concentrations to a 384 well microtiter plate. Fluorescence labeled NDP-alpha-MSH is dispensed at a single concentration, followed by the addition of membrane preparations. The plate is incubated for 60 to 90 min at room temperature.

The degree of fluorescence polarization is determined with a fluorescence polarization microplate reader.

### B. Functional Assay

A functional cellular assay, based on competition between unlabeled CAMP and a fixed quantity of fluorescence labeled CAMP for a limited number of binding sites on a CAMP specific antibody, is used to discriminate melanocortin receptor agonists from antagonists by fluorescence polarization.

HEK293 cells, expressing one of the human melanocortin receptors, are grown in 384 well microtiter plates and are stimulated at different concentrations of the test compound to effect CAMP production. Cells are lysed and a fluorescence labeled CAMP conjugate is dispensed, followed by the addition of anti-cAMP antibody used to detect the produced CAMP. The plate is read on a fluorescence polarization microplate reader and the amount of CAMP produced, as a response to a test compound, is compared to the production of CAMP resulting from stimulation with NDP-alpha-MSH.

The ability of a compound to block CAMP production, in response to NDP-alpha-MSH, is measured to define antagonistic activity of a test compound. Percent inhibition is determined by comparing the amount of CAMP produced in the presence to that produced in the absence of test compound.

### C. In Vivo Food Intake Models

### 1. Spontaneous Feeding Paradigm

Food intake in rats is measured after i.p. or p.o. administration of the test compound (see e.g. Chen, A.S. et al. Transgenic Res 2000 Apr;9(2):145-54).

### 2. Model of LPS and Tumor-Induced Cachexia

Prevention or amelioration of cachexia, induced by either lipopolysaccharide (LPS) administration or by tumor growth, is determined upon i.p. or p.o. administration of test compounds to rats (see e.g. Marks, D.L.; Ling, N and Cone, R.D. Cancer Res 2001 Feb 15;61 (4):1432-8).

### D. Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position, with the penile sheath retracted, for 15 - 30 minutes. Day 4, the animals are restrained in the supine position, with the penile sheath retracted, until penile responses are observed. Some animals require additional days of conditioning before they are completely acclimated to the procedures, non-responders are removed from further evaluation. After any handling or evaluation, animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur, spontaneously, within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and/or vehicle evaluations are conducted to determine how, and if, an animal will respond. Some animals have a long duration until the first response, while others are non-responders altogether. During this baseline evaluation, latency to first response and number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired 2 tailed t-tests to compared baseline and/or vehicle evaluations, to drug treated evaluations, for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays relevant to female sexual receptivity include the behavioral model of lordosis and direct observations of copulatory activity. There is also an urethrogenital reflex model in anesthetized spinally transected rats for measuring orgasm in both male and female rats. These and other established animal models of female sexual dysfunction are described in McKenna KE et al, A Model For The Study of Sexual Function In Anesthetized Male And Female Rats, Am. J. Physiol. (Regulatory Integrative Comp. Physiol 30): R1276-R1285, 1991; McKenna KE et al, Modulation By Peripheral Serotonin of The Threshold For Sexual Reflexes In Female Rats, Pharm. Bioch. Behav., 40:151-156, 1991; and Takahashi LK et al, Dual Estradiol Action In The Diencephalon And The Regulation of Sociosexual Behavior In Female Golden Hamsters, Brain Res., 359:194-207, 1985.

Representative compounds of the present invention were tested and found to bind to the melanocortin-4 receptor. These compounds were generally found to have IC50 values less than 2 µM. Representative compounds of the present invention were also tested in the functional assay and found generally to activate the melanocortin-4 receptor with EC50 values less than 1 µM.

### Examples of a Pharmaceutical Composition

As a specific embodiment of an oral composition of a compound of the present invention, 35 mg of Example 4 is formulated with sufficient finely divided lactose to provide a total amount of 580 to 590 mg to fill a size 0 hard gelatin capsule.

As another specific embodiment of an oral composition of a compound of the present invention, 50 mg of Example 46 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein, without departing from the spirit and scope of the invention. For example, effective dosages, other than the preferred doses as explained above, may be applicable as a consequence of the specific pharmacological responses observed and may vary, depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound of structural formula (I): 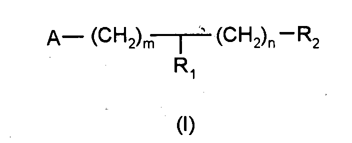 or a pharmaceutically acceptable salt or a solvate thereof, wherein
R₁ is:
(D)-aryl or (D)-heteroaryl,
wherein aryl and heteroaryl are unsubstituted or substituted;
R₂ is: 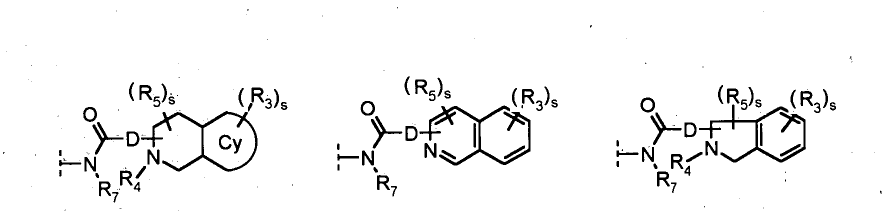 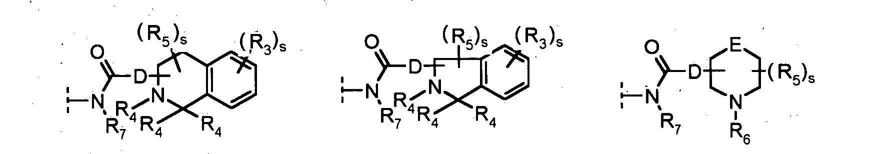 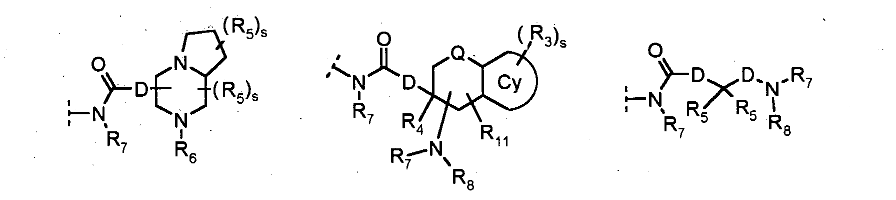 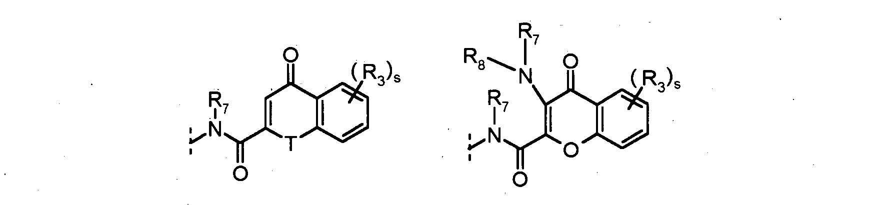 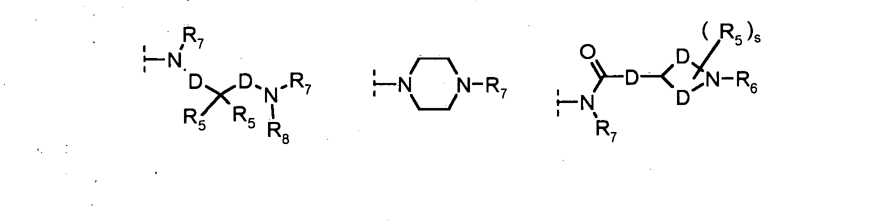
A is: 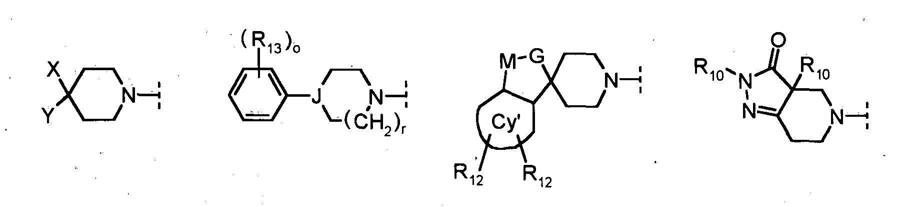
each R₃ is independently:
hydrogen,
halo,
alkyl,
haloalkyl,
(D)-cycloalkyl,
(D)-aryl (wherein aryl is phenyl or naphthyl),
(D)-heteroaryl,
(D)-heterocyclyl (wherein heterocyclyl excludes a heterocyclyl containing a single nitrogen), and
wherein aryl, heteroaryl, heterocyclyl, alkyl and cycloalkyl is unsubstituted or substituted;
each R₄ is independently:
hydrogen,
alkyl,
C(O)-alkyl,
SO₂alkyl,
SO₂aryl,
(D)-aryl or
(D)-cycloalkyl;
each R₅ is independently:
hydrogen,
alkyl,
(D)-aryl,
(D)-heteroaryl,
(D)-N(R₇)₂,
(D)-NR₇C(O)-alkyl,
(D)-NR₇SO₂-alkyl,
(D)-SO₂N(R₇)₂,
(D)-(O)_{q}-alkyl,
(D)-(O)_{q}(D)-NR₇COR₇,
(D)-(O)_{q}(D)-NR₇SO₂R₇,
(D)-(O)_{q}-heterocyclyl or
(D)-(O)_{q}(alkyl)-heterocyclyl;
each R₆ is independently:
hydrogen,
alkyl,
(D)-phenyl,
C(O)-alkyl,
C(O)-phenyl,
SO₂-alkyl or
SO₂-phenyl;
R₇ and R₈ are each independently:
hydrogen,
alkyl or
(D)-cycloalkyl, or
R₇ and R₈ together with the nitrogen to which they are attached form a 5- to 8-membered ring optionally containing an additional heteroatom selected from O, S and NR₄,
wherein alkyl and cycloalkyl are unsubstituted or substituted;
R₁₀ is independently:
hydrogen,
alkyl,
(D)-aryl or
(D)-cycloalkyl;
R₁₁ is
hydrogen or
alkyl;
R₁₂ is:
hydrogen,
halo,
alkyl,
alkoxy,
C≡N,
CF₃ or
OCF₃;
R₁₃ is independently:
hydrogen,
hydroxy,
cyano,
nitro,
halo,
alkyl,
alkoxy,
haloalkyl,
(D)-C(O)R₁₅.
(D)-C(O)OR₁₅,
(D)-C(O)SR₁₅,
(D)-C(O)-heteroaryl,
(D)-C(O)-heterocyclyl,
(D)-C(O)N(R₁₅)₂,
(D)-N(R₁₅)₂,
(D)-NR₁₅COR₁₅,
(D)-NR₁₅CON(R₁₅)₂,
(D)-NR₁₅C(O)OR₁₅,
(D)-NR₁₅C(R₁₅)=N(R₁₅),
(D)-NR₁₅C(=NR₁₅)N(R₁₅)₂,
(D)-NR₁₅SO₂R₁₅,
(D)-NR₁₅SO₂N(R₁₅)₂,
(D)-NR₁₅(D)-heterocyclyl,
(D)-NR₁₅(D)-heteroaryl,
(D)-OR₁₅,
OSO₂R₁₅,
(D)-[O]_{q}(cycloalkyl),
(D)-[O]_{q}(D)-aryl,
(D)-[O]_{q}(D)-heteroaryl,
(D)-[O]_{q}(D)-heterocyclyl (wherein heterocyclyl excludes a heterocyclyl containing a single nitrogen when q=1),
(D)-SR₁₅,
(D)-SOR₁₅,
(D)-SO₂R₁₅ or
(D)-SO₂N(R₁₅)₂,
wherein alkyl, alkoxy, cycloalkyl, aryl, heterocyclyl and heteroaryl are unsubstituted or substituted;
each R₁₅ is independently:
hydrogen,
alkyl,
haloalkyl,
(D)-cycloalkyl,
(D)-aryl (wherein aryl is phenyl or naphthyl),
(D)-heteroaryl,
(D)-heterocyclyl (wherein heterocyclyl excludes a heterocyclyl containing a single nitrogen), and
wherein aryl, heteroaryl, heterocyclyl, alkyl and cycloalkyl is unsubstituted or substituted;
R₁₇ is independently:
R₁₀ or
(D)-heterocyclyl;
R₁₈ is independently:
R₁₀,
(D)-heteroaryl,
(D)-heterocyclyl,
(D)-N(Y)₂.
(D)-NH-heteroaryl or
(D)-NH-heterocyclyl,
wherein aryl, heteroaryl, alkyl, D, cycloalkyl and heterocyclyl are unsubstituted or substituted, or
two R₁₈ groups together with the atoms to which they are attached form a 5-to 8-membered mono- or bi-cyclic ring system optionally containing an additional heteroatom selected from O, S, NR₁₀, NBoc and NZ;
Cy is:
aryl,
5- or 6-membered heteroaryl,
5- or 6-membered heterocyclyl or
5- or 7-membered carbocyclyl;
Cy' is:
benzene,
pyridine or
cyclohexane,
X is:
alkyl,
(D)-cycloalkyl,
(D)-aryl,
(D)-heteroaryl,
(D)-heterocyclyl,
(D)-C=N,
(D)-CON(R₁₇R₁₇).
(D)-CO₂R₁₇,
(D)-COR₁₇,
(D)-NR₁₇C(O)R₁₇,
(D)-NR₁₇CO₂R₁₇,
(D)-NR₁₇C(O)N(R₁₇)₂.
(D)-NR₁₇SO₂R₁₇,
(D)-S(O)_{P}R₁₇,
(D)-SO₂N(R₁₇)(R₁₇),
(D)-OR₁₇,
(D)-OC(O)R₁₇,
(D)-OC(O)OR₁₇,
(D)-OC(O)N(R₁₇)₂,
(D)-N(R₁₇)(R₁₇) or
(D)-NR₁₇SO₂N(R₁₇)(R₁₇).
wherein aryl, heteroaryl, alkyl, D, cycloalkyl and heterocyclyl are unsubstituted or substituted;
Y is:
hydrogen,
alkyl,
(D)-cycloalkyl,
(D)-aryl,
(D)-heterocyclyl or
(D)-heteroaryl,
wherein aryl, heteroaryl, alkyl, D and cycloalkyl are unsubstituted or substituted;
Q is a bond, O, S(O)ᵤ, NR₆ or CH₂;
D is a bond or C₁ - C₄ alkyl;
E is O, S or NR₆;
G is D, CH-alkyl, O, C=O or SO₂, with the proviso that when G is O, the ring atom M is carbon;
J is N or CH;
M is CHCO₂Y, CHC(O)N(Y)₂, NSO₂R₁₈, CHN(Y)COR₁₈, CHN(Y)SO₂R₁₈, CHCH₂OY or CHCH₂heteroaryl;
T is O or NR₇;
n is 0 - 3;
m is 1 - 3;
o is 0 - 3;
p is 0 - 2;
q is 0 or 1;
r is 1 or 2;
s is 0 - 3;
u is 0 - 2.

2. The compound of claim 1, wherein
R₁ is (D)-aryl which may be substituted with one to three substituents independently selected from the group consisting of cyano, nitro, perfluoroalkoxy, halo, alkyl (D)-cycloalkyl, alkoxy and haloalkyl;
R₂ is: 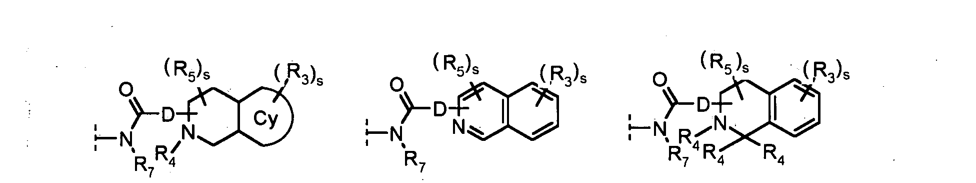 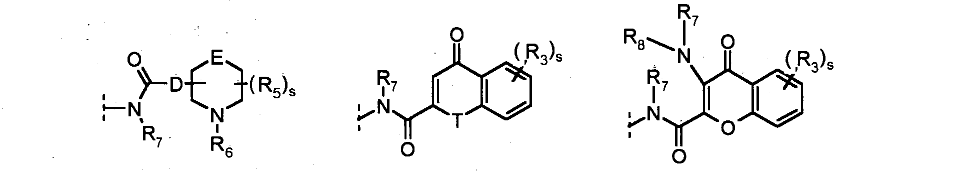 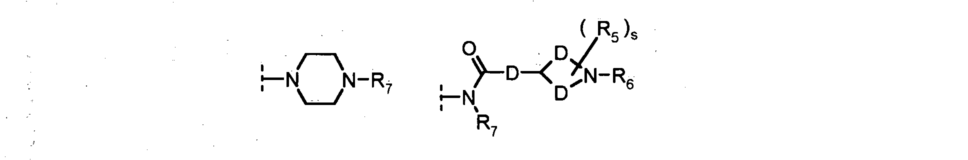
R₃ is independently:
hydrogen,
halo,
alkyl,
alkoxy,
haloalkyl or
(D)-cycloalkyl;
R₄ is:
hydrogen or
alkyl;
each R₅ is independently:
hydrogen,
alkyl,
(D)-aryl,
(D)-heteroaryl,
(D)-N(R₇)₂,
(D)-NR₇C(O)alkyl or
(D)-NR₇SO₂alkyl;
R₇ and R₈ are each independently:
hydrogen,
alkyl or
cycloalkyl, or
R₇ and R₈ together with the nitrogen to which they are attached form a 5- to 7-membered ring optionally containing an additional heteroatom selected from O, S and NR₄;
R₉ is:
alkyl,
OR₁₀,
(D)-aryl,
(D)-cycloalkyl,
(D)-heteroaryl and
halo;
R₁₂ is:
hydrogen,
halo,
alkyl,
alkoxy or
C=N;
R₁₃ is independently:
hydrogen,
hydroxy,
cyano,
nitro,
halo,
alkyl,
alkoxy,
haloalkyl,
(D)-N(R₁₅)₂
(D)-NR₁₅COR₁₅,
(D)-NR₁₅CON(R₁₅)₂,
(D)-NR₁₅C(O)OR₁₅,
(D)-NR₁₅C(R₁₅)=N(R₁₅),
(D)-NR₁₅C(=NR₁₅)N(R₁₅)₂,
(D)-NR₁₅SO₂R₁₅ or
(D)-NR₁₅SO₂N(R₁₅)₂;
each R₁₄ is independently:
hydrogen,
halo,
alkyl,
(D)-cycloalkyl,
alkoxy or
phenyl;
each R₁₅ is independently:
hydrogen,
halo,
alkyl,
(D)-cycloalkyl,
alkoxy or
phenyl;
each R₁₆ is independently:
hydrogen,
alkyl or
cycloalkyl;
X is:
alkyl,
(D)-cycloalkyl,
(D)-aryl,
(D)-heteroaryl,
(D)-heterocyclyl,
(D)-NHC(O)R₁₇,
(D)-CO₂R₁₇ or
(D)-CON(R₁₇R₁₇);
Y is:
hydrogen,
alkyl,
(D)-cycloalkyl,
(D)-aryl,
(D)-heterocyclyl or
(D)-heteroaryl;
Cy is:
aryl,
5- or 6-membered heteroaryl,
5- or 6-membered heterocyclyl or
5- to 7-membered carbocyclyl;
Cy' is benzene or pyridine;
D is a bond or C₁ - C₄-alkylene;
M is NSO₂R₁₈, CHN(Y)COR₁₈ or CHN(Y)SO₂R₁₈;
G is D or CH-alkyl;
T is NR₇;
n is 0 or 1;
m is 1 or 2;
r is 1;
s is 0, 1 or 2.

3. The compound of claims 1 or 2, wherein
R₁ is (D)-phenyl or (D)-naphthyl which may be substituted with one or two substituents independently selected from the group consisting of perfluoroalkoxy, halo, alkyl, alkoxy and haloalkyl;
R₂ is: 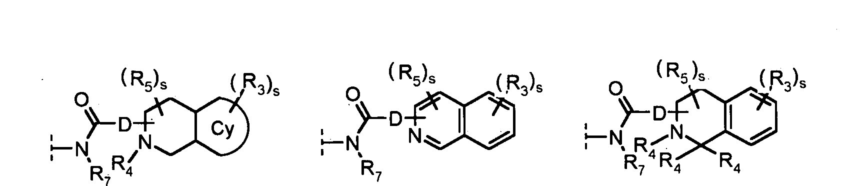 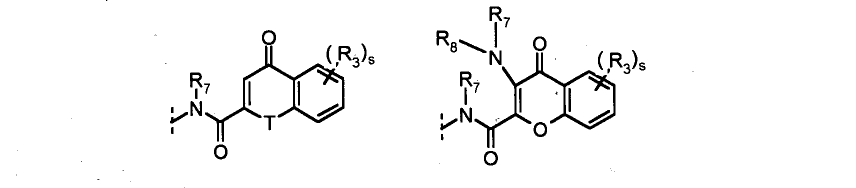 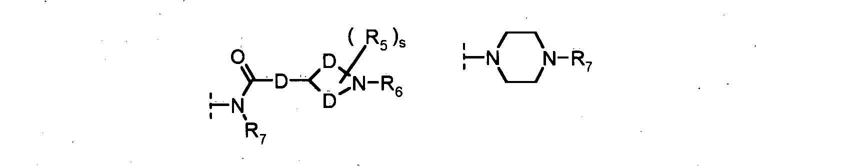
R₃ is hydrogen;
R₄ is hydrogen;
R₅ is hydrogen;
R₇ and R₈ are each independently:
hydrogen or
alkyl, or
R₇ and R₈ together with the nitrogen to which they are attached form a 5- to 6-membered ring optionally containing an additional oxygen atom;
R₁₂ is:
hydrogen,
halo or
C₁ - C₄ alkyl;
R₁₃ is independently:
cyano,
nitro,
halo,
alkyl,
(D)-N(R₁₅)₂,
(D)-NR₁₅COR₁₅,
(D)-NR₁₅CON(R₁₅)₂.
(D)-NR₁₅C(O)OR₁₅ or
(D)-NR₁₅SO₂R₁₅-,
each R₁₄ is independently:
hydrogen,
halo,
alkyl,
alkoxy or
phenyl;
each R₁₅ is independently:
hydrogen,
halo,
alkyl,
alkoxy or
phenyl;
X is:
alkyl,
(D)-cycloalkyl,
(D)-heterocyclyl,
(D)-NHC(O)R₁₇ or
(D)-CON(R₁₇R₁₇);
Y is:
hydrogen,
alkyl,
(D)-cycloalkyl or
(D)-heterocyclyl;
Cy is
aryl or
5- or 6-membered heteroaryl;
Cy' is benzene;
D is a bond or CH₂;
M is NSO₂R₁₈;
G is D;
s is 0 or 1.

4. The compound of any of claims 1 to 3, wherein
R₁ is (CH₂)-phenyl or (CH₂)-naphthyl which may be substituted with one to three halo atoms;
R₂ is: 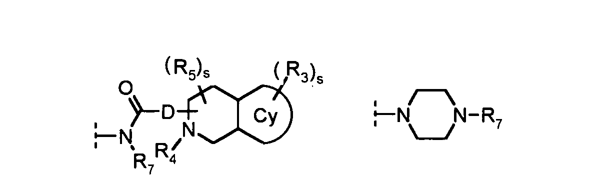 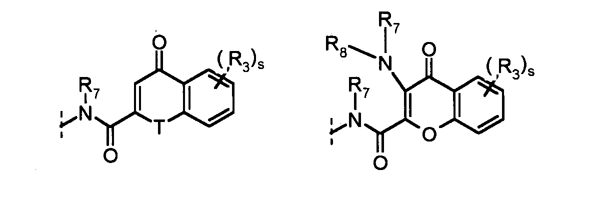
R₁₂ is hydrogen;
R₁₃ is independently:
cyano,
nitro,
halo or
(D)-NR₁₅COR₁₅;
X is:
C₁ - C₄ alkyl,
C₅ - C₇ cycloalkyl,
(D)-CON(R₁₇R₁₇) or
N-containing heterocyclyl;
Y is:
hydrogen,
C₁ - C₄ alkyl or
C₅ - C₇ cycloalkyl;
Cy is aryl;
G is CH₂.

5. The compound of any of claims 1 to 4 for use as a medicament.

6. The intermediate compound of structural formula (II): 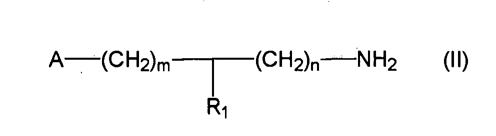 wherein A, R₁, m and n are as defined in claim 1
for use as a medicament.

7. Use of the compound of any of claims 1 to 4 for the preparation of a medicament for the treatment or prevention of disorders, diseases or conditions responsive to the inactivation or activation of the melanocortin-4 receptor.

8. Use of the intermediate compounds of claim 6 for the treatment or prevention of disorders, diseases or conditions responsive to the inactivation or activation of the melanocortin-4 receptor in a mammal.

9. Use according to claims 7 and 8 for the treatment or prevention of cancer cachexia.

10. Use according to claims 7 and 8 for the treatment or prevention of muscle wasting.

11. Use according to claims 7 and 8 for the treatment or prevention of anorexia.

12. Use according to claims 7 and 8 for the treatment or prevention of anxiety and/or depression.

13. Use according to claims 7 and 8 for the treatment or prevention of obesity.

14. Use according to claims 7 and 8 for the treatment or prevention of diabetes mellitus.

15. Use according to claims 7 and 8 for the treatment or prevention of male or female sexual dysfunction.

16. Use according to claims 7 and 8 for the treatment or prevention of erectile dysfunction.

17. A pharmaceutical composition which comprises a compound of any of claims 1 to 4 and a pharmaceutically acceptable carrier.

18. A pharmaceutical composition which comprises an intermediate compound of claim 6 and a pharmaceutically acceptable carrier.
